# EUROPÄISCHE PATENTSCHRIFT

(11) **EP 1 492 755 B1**
(45) Veröffentlichungstag und Bekanntmachung des Hinweises auf die Patenterteilung: **23.12.2009**
(21) Anmeldenummer: 03709791.2
(22) Anmeldetag: 17.03.2003
(51) Int. Cl.: C07C 51/43, C07C 57/04

(54) **(METH)ACRYLSAEUREKRISTALL UND VERFAHREN ZUR HERSTELLUNG UND AUFREINIGUNG VON WAESSRIGER (METH)ACRYLSAEURE**
(METH)ACRYLIC ACID CRYSTAL AND METHOD FOR THE PRODUCTION AND PURIFICATION OF AQUEOUS (METH)ACRYLIC ACID
CRISTAL D'ACIDE (METH)ACRYLIQUE ET PROCEDE POUR PRODUIRE ET PURIFIER DE L'ACIDE (METH)ACRYLIQUE AQUEUX

(30) Priorität: 15.03.2002 DE 10211686
(43) Veröffentlichungstag der Anmeldung: 05.01.2005
(73) Patentinhaber: Evonik Stockhausen GmbH, 47805 Krefeld (DE)
(72) Erfinder: NORDHOFF, Stefan, 45657 Recklinghausen (DE); BALDUF, Torsten, 45772 Marl (DE); BUB, Günther, 45772 Marl (DE); FORNIKA, Roland, 48249 Dülmen (DE); MOSLER, Jürgen, 44577 Castrop-Rauxel (DE); RATHKE, Thomas, Mandeville, LA 70448 (US); KOBUS, Axel, 44797 Bochum (DE); THONG, Dennis, 45772 Marl (DE)
(86) Internationale Anmeldenummer: PCT/EP2003/002753
(87) Internationale Veröffentlichungsnummer: WO 2003/078378

(56) Entgegenhaltungen:
- EP-A- 0 616 998
- DE-A- 19 740 252
- US-A- 4 780 568

## Beschreibung

Die vorliegende Erfindung ist auf ein Verfahren zur Aufreinigung einer (Meth)Acrylsäure beinhaltenden Zusammensetzung, eine Vorrichtung zur Herstellung von reiner (Meth)Acrylsäure, eine Vorrichtung zur Polymerisation von (Meth)Acrylsäure), (Meth)Acrylsäure bzw. Polymere, erhältlich aus diesen Verfahren, deren Verwendung und diese beinhaltende Stoffe gerichtet.

"(Meth)Acrylsäure" wird in diesem Text für die Verbindungen mit den Nomenklaturnamen "Methacrylsäure" und "Acrylsäure" verwendet. Von beiden Verbindungen ist die Acrylsäure erfindungsgemäß bevorzugt.

Die Aufarbeitung von (Meth)Acrylsäure zu hohen Reinheiten von bis zu >99,9 Gew.-% ist für deren Einsatz in Polymeren häufig erwünscht. So wird beispielsweise im Hygienebereich im Falle von Superabsorbern auf Basis von Polyacrylaten gefordert, dass bestimmte Nebenprodukte nur unterhalb der Nachweisgrenze vorhanden sein dürfen.

Als eine Alternative zur Herstellung von hochreinen organischen Substanzen ist die Kristallisation zu nennen. Technisch kommen dabei insbesondere zwei Verfahren zur Anwendung, die Suspensionskristallisation und die Schichtkristallisation (Wintermantel et al., Chem. Ing. Tech. 1991, 63,881-891; Steiner et al, Chem. Ing. Tech. 1985, 57, 91-102).

Oft reicht allerdings ein Kristallisationsschritt alleine nicht aus, um Nebenprodukte hinreichend gut aus oder von den Kristallen zu entfernen, da Mikroeinschlüsse von Mutterlaugen oder Einbau von Verunreinigungen an Kristallfehlstellen etc. unter endlichen Kristallwachstumsbedingungen nicht auszuschließen sind. Auch das Anhaften von Mutterlauge auf dem Kristall kann die Reinheit der Produkte verschlechtern.

Aus diesem Grund werden die erzeugten Kristalle häufig, insbesondere im Falle einer Kristallsuspension, nach der Trennung von der Mutterlauge mit Waschflüssigkeiten gewaschen und/oder die Kristalle werden bei Schicht- bzw. Suspensionskristallisation einem Schwitzprozess unterzogen, bei dem Verunreinigungen jeglicher Art ggf. abgereichert werden können. Kontinuierlich kann ein solcher Prozess in sogenannten Waschkolonnen durchgeführt werden. Eine Übersicht bietet hier die Dissertation von Poschmann (Zur Suspensionskristallisation organischer Schmelzen und Nachbehandlung der Kristalle durch Schwitzen und Waschen, Diss. Uni. Bremen, Shaker Verlag, Aachen 1996).

Die EP0616998 offenbart ein Verfahren zur Herstellung von >99,9 Gew.-%iger Acrylsäure ausgehend von vorgereinigtem Produkt mit einem Acrylsäuregehalt von 97,771 Gew.-%. Der Reinigungseffekt wird durch ein Zusammenwirken von dynamischen und statischen Schichtkristallisationsverfahren erreicht. Als finales Kristallisationsorgan findet hier eine sogenannte Fallfilmkristallisation Anwendung. Das Betreiben einer derartigen Anlage ist nur diskontinuierlich möglich und bedingt durch die vielen Prozesszyklen, die zum Erhalt der entsprechenden Reinheiten notwendig sind, einen hohen apparativen wie logistischen Aufwand und einen vergleichsweise hohen Energieaufwand.

Aus WO99/14181 ist bekannt, Roh-(Meth)Acrylsäure zur Reinigung in einem ersten Schritt zu kristallisieren und in einem zweiten Schritt ggf. mittels Waschkolonnen aufzuarbeiten. Das dort offenbarte Verfahren geht direkt von den Kondensationsprodukten der katalytischen Gasphasenoxidation zur Erzeugung von (Meth)Acrylsäure aus. Bei diesem Verfahren wird beschrieben, dass die nach dem Waschen und Abtrennen der Kristalle entstehende Mutterlauge in die Kondensationsstufe zurückgeführt wird. Mittels dieses Verfahrens erhielt man aus 90,972 Gew.-%iger Acrylsäure ein Produkt mit einer Reinheit von 98,8816 Gew.-%. Dies ist für manche technischen Anwendungen jedoch nicht ausreichend. So bildet gerade der Gehalt an Inhibitoren und Aldehyden in der reinen (Meth)Acrylsäure eine kritische Größe, die bei Überschreitung z.B. in den nachgeschalteten Polymerisationsverfahren für Nachteile sorgt.

In US-A-4780568 wird ein Verfahren zur Reinigung von Methacrylsäure beschrieben, welches von einer im wesentlichen wasserfreien Zusammensetzung als Ausgangszubereitung ausgeht.

Von Nienrood et al. wurde beschrieben, dass Acrylsäure durch Suspensionskristallisation und anschließende Behandlung in einer hydraulischen Waschkolonne gut aufgereinigt werden kann (sogenanntes TNO-Verfahren; Proc. Bremer International Workshop on Industrial Crystallization, Bremen, 1994, Hrsg.: J. Ulrich, S.4-11; Purification Potential of Suspension Growth Melt Crystallization, Proc. 4th International Workshop on Crystal Growth of Organic Materials, Bremen, 1997, Hrsg.: J. Ulrich, Aachen Shaker Verlag, S.139-145). Die bei diesen Untersuchungen eingesetzte Acrylsäure wurde von Aldrich bezogen und hatte eine Reinheit von 99,75 Gew.-%. Sie ließ sich mittels dieses Verfahrens auf eine Reinheit von 99,97 Gew.-% aufreinigen. Nicht offenbart wurde jedoch der Einsatz von Acrylsäure mit geringeren Reinheiten.

Bei bisherigen Verfahren zur Herstellung von (Meth)Acrylsäure wird üblicherweise die in einem (Meth)Acrylsäurereaktor gewonnene und anschließend in einem Quenchabsorber in Wasser gelöste (Meth)Acrylsäure einem aufwendigen Destillationsverfahren, in denen zum Teil der Einsatz von Schleppmitteln wie Toluol erforderlich ist, unterzogen, um anschließend durch einen Kristallisationsvorgang hohe Reinheitsgrade zu erzielen. Nachteilig von Destillationsverfahren für (Meth)Acrylsäure ist, dass die (Meth)Acrylssäure während des Destillationsvorgangs thermisch belastet wird, wodurch sich, z.B. durch eine teilweise (Vor)Polymerisation, ihre Eigenschaften verändern.

Allgemein liegt der vorliegenden Erfindung die Aufgabe zu Grunde, die sich aus dem Stand der Technik ergebenden Nachteile durch die Bereitstellung geeigneter technischer Lehren zu überwinden.

Gemäß einer weiteren erfindungsgemäßen Aufgabe soll ein Verfahren zur Erzeugung hochreiner (Meth)Acrylsäure aus einem verunreinigten Roh-(Meth)Acrylsäurestrom aus einem Verfahren zur Herstellung von (Meth)Acrylsäure angegeben werden. Dabei sollte das Verfahren in Bezug auf die (Meth)Acrylsäure schonend, im technischen Maßstab gut anwendbar und deshalb vom ökonomischen wie ökologischen Standpunkt den Verfahren des Standes der Technik überlegen sein. Insbesondere sind in diesem Zusammenhang ein hervorragendes Aufreinigungsvermögen, bei Einhaltung hoher ökologischer und ökonomischer Anforderungen zu nennen.

Zudem lag eine weitere erfindungsgemäße Aufgabe darin, eine Vorrichtung zur Erzeugung hochreiner (Meth)Acrylsäure zur Verfügung zu stellen, die eine Aufreinigung von verunreinigter (Meth)Acrylsäure zu höchster Reinheit bei einem geringen Energieaufwand und störungsfreien und umweltschonenden Betrieb ermöglicht.

Ferner lag der Erfindung die Aufgabe zu Grunde, ein Verfahren und eine Vorrichtung zur Verfügung zu stellen, wobei bei der Herstellung und insbesondere bei der Aufreinigung von (Meth)Acrylsäure die Gefahr, das es zu unkontrollierter Polymerisation von (Meth)Acrylsäure kommt, verringert wird.

Außerdem bestand eine erfindungsgemäße Aufgabe darin, die zur Stabilisierung der (Meth)Acrylsäure zwischen Herstellung und Weiterverarbeitung der (Meth)Acrylsäure notwendige Menge an Stabilisatoren zu verringern.

Auch war es Aufgabe, eine weitere Verwendung der Vorrichtung zur Herstellung der (Meth)Acrylsäure bzw. eine ihrer Komponenten anzugeben, in denen die Gefahr der ungewünschten und unkontrollierten Polymerisation der (Meth)Acrylsäure gering ist.

Ferner lag eine Aufgabe darin, ein Verfahren und eine Vorrichtung zur Verfügung zu stellen, mit der wässrige Lösungen von möglichst reiner (Meth)Acrylsäure hergestellt werden können. Diese Aufgabe stellt sich insbesondere vor dem Hintergrund, dass bei der Herstellung von (Meth)Acrylsäure beinhaltenden Polymeren oftmals wässrige (Meth)Acrylsäuren in Lösungs- Suspensions- oder Emulsionspolymerisationen eingesetzt werden. Hierbei ist es von Interesse, dass der Energieaufwand, der mit Lösungsmittelwechseln in den einzelnen Syntheseschritten von der Monomersynthese bis zur Polymerisation verbunden ist, möglichst gering gehalten wird.

Zudem lag eine erfindungsgemäße Aufgabe darin, eine wässrige (Meth)Acrylsäure-Phase zur Verfügung zu stellen, die eine vergleichsweise hohe Wasserkonzentration bei möglichst hoher (Meth)Acrylsäure-Reinheit aufweist. Derartige wässrige Acrylsäurephasen sind insofern vorteilhaft, weil diese ohne einen weiteren Verdünnungsschritt unmittelbar in die wässrige Polymerisation von absorbierenden Polymeren eingesetzt werden können. Auf diese Weise wird die bisher übliche destillative Abtrennung von Wasser, gefolgt von der Aufreinigung der Acrylsäure und einem erneuten Zusatz von Wasser zur Polymerisation des wasserabsorbierenden Polymers um einen Schritt verkürzt.

Diese Aufgaben werden gelöst durch ein Verfahren zur Aufreinigung einer Zusammensetzung beinhaltend (Meth)Acrylsäure, mindestens eine Verunreinigung und Wasser, wobei die Zusammensetzung einen Wassergehalt im Bereich von 0,55 bis 90, vorzugsweise von 7 bis 50 und besonders bevorzugt von 10 bis 25, oder auch von 10 bis 85, vorzugsweise von 15 bis 80, besonders bevorzugt 25 bis 75 Gew.-%, jeweils bezogen auf die Zusammensetzung, aufweist zu einer gereinigten Phase, beinhaltend (Meth)Acrylsäure und mindestens eine Verunreinigung, wobei in der gereinigten Phase die Menge an mindestens einer Verunreinigung weniger als 7 Gew.-%, vorzugsweise weniger als 5 Gew.-%, bevorzugt weniger als 3 Gew.-% und besonders bevorzugt weniger als 1 Gew.-%, bezogen auf die (Meth)Acrylsäure in der gereinigten Phase, beinhaltet, aufweisend eine Verfahrensstufe, welche folgende Verfahrensschritte umfasst:
a) (Meth)Acrylsäure wird aus der Zusammensetzung unter Bildung einer Suspension, beinhaltend eine Mutterlauge und (Meth)Acrylsäurekristalle, auskristallisiert;
b) (Meth)Acrylsäurekristalle werden von der Mutterlauge abgetrennt;
c) mindestens ein Teil der abgetrennten (Meth)Acrylsäurekristalle wird zu einer Schmelze aufgeschmolzen; und
d) ein Teil der Schmelze wird dem Schritt a) oder dem Schritt b), vorzugsweise Schritt b), zurückgeführt und wobei der nicht zurückgeführte Teil der Schmelze als eine abgetrennte (Meth)Acrylsäure vorliegt.

Die abgetrennte (Meth)Acrylsäure bildet, sofern nicht weitere Verfahrenstufen oder -Schritte auf d) folgen, einen Bestandteil der gereinigten Phase.

Gemäß einer Ausführungsform des erfindungsgemäßen Verfahrens weist die Zusammensetzung als eine wasserreiche Zusammensetzung eine Konzentration von (Meth)Acrylsäure mit bis zu 10 Gew.-% Verunreinigungen im Bereich von 45 bis 80 Gew.-% und Wasser im Bereich von 20 bis 55 Gew.-%, jeweils bezogen auf die Zusammensetzung, auf. Die wässrigen Zusammensetzungen können aus einem Quenchabsorber stammen. Weiterhin kann es sich bei den wässrigen Zusammensetzungen um eine (Meth)Acrylsäure abgereicherte Mutterlauge oder das Filtrat aus einer Kristallisation handeln, deren (Meth)Acrylsäure weiterhin aufgereinigt werden soll.

Im Fall der wasserreichen Zusammensetzung weist die sich in Schritt a) bildende Suspension vorzugsweise neben (Meth)Acrylsäurekristallen noch Wasserkristalle auf. Es ist weiterhin bevorzugt, dass im Fall der wasserreichen Zusammensetzung die Schmelze neben der abgetrennten (Meth)Acrylsäure im Bereich von 10 bis 90 Gew.-%, bevorzugt von 15 bits 70 Gew.-% und besonders bevorzugt von 25 bis 55 Gew.-%, bezogen auf die Schmelze, Wasser aufweist.

Gemäß einer anderen Ausführungsform des erfindungsgemäßen Verfahrens weist die Zusammensetzung als eine wasserarme Zusammensetzung eine Konzentration von (Meth)Acrylsäure mit bis zu 10 Gew.-% Verunreinigungen im Bereich von mehr als 80 Gew.-% und Wasser im Bereich von unter 20 bis 0,55 Gew.-%, jeweils bezogen auf die Zusammensetzung, auf. Diese wasserarmen Zusammensetzungen stammen vorzugsweise aus thermischen Abreicherung, bevorzugt der Destillation, von (Meth)Acrylsäure oder aus einem vorgeschalteten Kristallisationsschritt, der vorzugsweise dem erfindungsgemäßen Verfahren entspricht.

In einer weiteren Ausführungsform des erfindungsgemäßen Verfahrens ist es bevorzugt, dass die gereinigte Phase als eine wasserarme Phase mindestens 30 Gew.-%, vorzugsweise mindestens 55 Gew.-% und besonders bevorzugt mindesten 75 Gew.-% (Meth)Acrylsäure, sowie weniger als 5 Gew.-%, vorzugsweise weniger als 2 Gew.-%, besonders bevorzugt weniger als 1 Gew.-% und darüber hinaus bevorzugt weniger als 0,5 Gew.-% Wasser, jeweils bezogen auf die gereinigte Phase, aufweist.

In einer anderen Ausführungsform des erfindungsgemäßen Verfahrens ist es bevorzugt, dass die gereinigte Phase als wasserreiche Phase mindestens 20 Gew.-%, vorzugsweise mindestens 30 Gew.-% und besonders bevorzugt mindesten 70 Gew.-% (Meth)Acrylsäure, sowie im Bereich von 5 bis 80 Gew.-%, vorzugsweise im Bereich von 10 bis 70 Gew.-%, besonders bevorzugt im Bereich von 15 bis 30 Gew.-% Wasser, jeweils bezogen auf die gereinigte Phase, aufweist.

Die wasserarme Phase kann beispielsweise einer lösungsmittelfreien Polymerisation oder einer Polymerisation zugeführt werden, in der das Lösungsmittel erst vor oder während der Polymerisation zugesetzt wird. Hingegen kann die wasserreiche Phase einer Lösungs-, Suspensions- oder Emulsionspolymerisation, in der Wasser als ein Lösungsmittel verwendet wird, sofort zugesetzt werden.

Durch geeignete Kombination des erfindungsgemäßen Verfahrens ggf. in mehreren Stufen, lässt sich (Meth)Acrylsäure in gewünschten Reinheiten erhalten.

So kann die aus der Kristallisation von einer wässrigen Zusammensetzung erhaltene (Meth)Acrylsäure als wasserarme (Meth)Acrylsäure weiter aufreinigt werden. Die dabei als wässrige Zusammensetzung anfallende Mutterlauge kann die nach dem erfindungsgemäßen Verfahren zu einer wasserarmen oder wässrigen Phase weiterverarbeitet werden.

In dem erfindungsgemäßen Verfahren ist es bevorzugt in dem Schritt a) (Meth)Acrylsäure zumindest teilweise, vorzugsweise zu mindestens 5 Gew.-%, besonders bevorzugt zu mindestens 40 Gew.-% und darüber hinaus bevorzugt zu mindestens 70 Gew.-% der Kristalle, zu einem Kristall mit einer Kristallstruktur mit einer Oberfläche mit mindestens einer an der Oberfläche befindlichen Ausnehmung auszukristallisieren, wobei die Kristallstruktur ein orthorombisches Bravais-Kristallgitter mit Raumgruppe Ibam, kristallographische Daten a=9,952 A, b=11,767 A und c=6,206 A aufweist (vgl. R Boese, D. Blaser, I. Steller, R. Latz, A. Baumen Acta Crystallogr., Sect (Cr. Str. Comm.), 55, 9900006, 1999).

Weiterhin ist es in dem erfindungsgemäßen Verfahren bevorzugt, dass in dem Schritt a) die Mutterlauge zumindest 95 Gew.-% (Meth)Acrylsäure und Wasser aufweist, wobei die Wasserkonzentration der Mutterlauge im Bereich von 10 und 90 Gew.-%, insbesondere von 15 und 70 Gew.-%, vorzugsweise von 20 und 40 Gew.-% liegt.

Besonders bevorzugt ist es, dass die Kristalle in ihrer Längsrichtung eine röhrenförmige Ausnehmung aufweisen, wobei diese vorzugsweise hantelförmig ist, wobei auf den Stirnflächen der annähernd quaderförmigen Kristalle die Öffnungen der röhrenförmigen Ausnehmung größer sind als in der parallel zu den Stirnflächen liegenden Schnittfläche des Kristalls.

Ferner ist es in dem erfindungsgemäßen Verfahren bevorzugt, dass die Kristallisation in einer Suspension und nicht durch Bildung von Schichten erfolgt, in der mehrere Einzelkristalle miteinander zu im Vergleich zu einer Kristallsuspension starren, kompakten Schicht verbunden sind. Dieses wird vorzugsweise dadurch erreicht, dass die Kristallisation nicht an einer entsprechend temperierten Fläche - beispielsweise der Wandung eines Schichtkristallers - unter Ausbildung einer Kristallschicht erfolgt. Vielmehr sind die sich in der Suspension bildenden Kristalle in der diese beinhaltenden flüssigen Phase wie der Mutterlauge frei beweglich.

Die vorstehend beschriebenen Ausnehmung erschwert auf vorteilhafte Weise, dass Verunreinigungen, wie z.B. Mutterlauge, im Inneren des Kristalls eingeschlossen werden. Durch die offenen Ausnehmungen können die Verunreinigungen abfließen bzw. heraus gewaschen werden.

Selbst ein hoher Wassergehalt der (Meth)Acrylsäure führt nicht zu einer sonst üblichen erhöhten Anzahl von Einschlüssen, sondern zu an beiden Seiten geöffneten Hohlräumen. Wird eine wasserhaltige Suspension mit solchen Kristallen Fest-Flüssig-getrennt, kann die anhaftende Mutterlauge vorteilhaft abfließen. Dieser Reinigungseffekt kann insbesondere dazu genutzt werden, um die Herstellung von (Meth)Acrylsäure erheblich zu vereinfachen und die Qualität der so hergestellten (Meth)Acrylsäure zu verbessern und auf diesem Wege reine ggf. wässrige (Meth)Acrylsäuren zu erhalten.

Durch das Quenchen der durch katalytische Gasphasenreaktion gewonnen (Meth)Acrylsäure mit Wasser in einem Quenchabsorber fallen wässrige (Meth)Acrylsäuren an, deren Wassergehalt in der Regel im Bereich von 10 und 90, vorzugsweise von 11 und 50 besonders bevorzugt von 12 bis 40 Gew.-% und darüber hinaus bevorzugt von 25 bis 35 Gew.-%, bezogen auf die gesamte Menge von Wasser und (Meth)Acrylsäure, liegt. Derartige Wasserkonzentrationen mussten bislang fast vollständig reduziert werden, um durch Kristallisation eine ausreichende Reinheit der (Meth)Acrylsäure zu erzielen. Hierzu wurde insbesondere mindestens ein Destillationsschritt notwendig, der durch die thermische Belastung die Qualität der (Meth)Acrylsäure aufgrund der einsetzenden Polymerisation beeinträchtigt.

In einer Ausgestaltung beträgt das Volumen der Ausnehmung mindestens 5 Vol.-%, insbesondere mindestens 10 Vol.-%, vorzugsweise 20 Vol.-% , besonders bevorzugt 50 Vol.-% des Volumens des Kristalls. Durch derartig große Volumina der Ausnehmungen wird sichergestellt, dass im Inneren des Kristalls nur vergleichsweise wenig Verunreinigungen auftreten können.

In einer weiteren Ausgestaltung der Erfindung weist der Kristall mindestens einen Einschluss auf, wobei die Summe der Volumina der Einschlüsse weniger als 30 Vol.-%, vorzugsweise weniger als 15 Vol.-% und besonders bevorzugt weniger als 5 Vol.-% sowie darüber hinaus bevorzugt weniger als 1 Vol.-%, des Volumens des Kristalls beträgt. Durch das kleine Volumen der Einschlüsse an verunreinigter Mutterlauge werden Verunreinigungen, die nicht abfließen oder durch einen Waschprozess entfernt werden können, der (Meth)Acrylsäure bezogen auf das Kristallvolumen begrenzt.

In einer anderen Ausgestaltung weist der Kristall eine Länge auf, die im Mittel zwischen 0,001 und 5 mm, insbesondere zwischen 0,05 und 0,5 mm, vorzugsweise zwischen 0,1 und 0,2 mm, liegt. Die Länge wird aus 500 zufällig ausgewählten Kristallen aus über einen Lichtmikroskop gewonnen photographischen Aufnahmen über Bildanalysesystem bestimmt. Hierzu wird ein Bildanalysesystem, bestehend aus einem Lichtmikroskop mit angeschlossener CCD-Kamera und einer PC-Auswerteeinheit eingesetzt, wobei ein PC-Programm der Fa. Soft Imaging System (SIS, V3.1)Verwendung findet.

Die in dem erfindungsgemäßen Verfahren eingesetzte Zusammensetzung, insbesondere zum Auskristallisieren eines erfindungsgemäßen Kristalls besteht zumindest zu 60 Gew.-%, vorzugsweise 80 Gew.-% und besonders bevorzugt 95 Gew.-% aus (Meth)Acrylsäure und Wasser, der Rest sind sonstige Stoffe beispielsweise bei der Synthese von (Meth)Acrylsäure anfallende Nebenprodukte, wobei die Wasserkonzentration zwischen 0,5 und 60 Gew.-%, insbesondere zwischen 5 und 40 Gew.-%, vorzugsweise zwischen 15 und 35 Gew.-%, liegt.

Mit diesem Verfahren gelangt man in überraschender Weise, dafür aber nicht minder vorteilhaft, zu hoch reinen Produkten, die sich auch für den Einsatz in Polymeren, z.B. für den Hygienebereich, eignen.

Das Verfahren arbeitet vorzugsweise kontinuierlich. Zur Abtrennung der (Meth)Acrylsäurekristalle von der Mutterlauge wird vorteilhafterweise eine Waschkolonne verwendet. Dazu weist die Waschkolonne einen Trennbereich auf, in dem die (Meth)Acrylsäurekristalle gewaschen werden. Für den erfolgreichen Betrieb einer Waschkolonne ist es vorteilhaft, dass die zu waschenden Kristalle hart genug sind und eine bestimmte enge Größenverteilung aufweisen, um eine entsprechende Porosität und Stabilität des entstehenden gepackten oder ungepackten Filterbettes zu gewährleisten.

Weiterhin ist es bevorzugt, dass das erfindungsgemäße Verfahren durch einen Kristallisationsaufwand (Wellinghoff, Wintermantel, CIT 63 (1991) 9, S. 805 Kap. 3.2.1) von 1 bis 4,5, vorzugsweise weniger 1 bis 3,5 und besonders bevorzugt von 1 bis 2,5 sowie darüber hinaus bevorzugt von 1 bis 1,5 gekennzeichnet ist.

Das erfindungsgemäße Verfahren kann mit verunreinigter Roh-(Meth)Acrylsäure betrieben werden, die <99,5 Gew.-% (Meth)Acrylsäure aufweist. Bevorzugt hat die eingesetzte Roh-(Meth)Acrylsäure eine Reinheit von 50 Gew.-% bis 95 Gew.-%, vorzugsweise 75 Gew.-% bis 90 Gew.-% an (Meth)Acrylsäure.

Darüber hinaus bevorzugt kann man das kondensierte Gemisch einer katalytischen Gasphasenoxidation zur Herstellung von (Meth)Acrylsäure direkt als Zusammensetzung in das erfindungsgemäße Verfahren, vorzugsweise in in den Schritt a), einsetzen.

Dazu wird (Meth)Acrylsäure, meist von einem entsprechenden Olefin ausgehend, in einem Reaktor generiert, dann wird sie in einem Quenchabsorber in wässerige Zusammensetzung gebracht, anschließend kann die (Meth)Acrylsäure in einer Destillationsvorrichtung zu einer wasserarmen Zusammensetzung destilliert werden. Der so hergestellte Roh-(Meth)Acrylsäurestrom wird der Aufreinigungsvorrichtung zugeführt. Das Verfahren erlaubt die Herstellung sehr reiner (Meth)Acrylsäure aus vergleichsweise verunreinigter Roh-(Meth)Acrylsäure.

Bei der Kristallisation sind als Kristallisationsmittel solche zu verwenden, die es gestatten, den erfindungsgemäßen Aufreinigungsprozeß kontinuierlich zu gestalten. Vorzugsweise kommt die Suspensionskristallisation zum Einsatz. Diese kann vorteilhafterweise in einem Rührkesselkristallisator, Kratzkristallisator, Kühlscheibenkristallisator, Kristallisierschnecke, Trommelkristallisator, Rohrbündelkristallisator o. dgl. durchgeführt werden. Insbesondere können die in WO99/14181 genannten Kristallisationsvarianten für den genannten Zweck herangezogen werden. Von besonderem Vorteil sind hier wiederum solche Kristallisatoren, die kontinuierlich betrieben werden können. Vorzugsweise sind dies die Kühlscheibenkristallisatoren oder der Kratzkühler (Diss. Poschmann, S. 14). Ganz besonders bevorzugt wird zur Kristallisation ein Kratzkühler eingesetzt.

Im Prinzip kann für das erfindungsgemäße Verfahren jedwede Waschkolonne eingesetzt werden, die die kontinuierliche Fahrweise der erfindungsgemäßen Aufreinigung zulässt. Bei einer üblichen Ausführungsform wird die Suspension in einer hydraulischen Waschkolonne im oberen Teil der Kolonne aufgegeben; die Mutterlauge wird über ein Filter aus der Kolonne abgezogen, wodurch sich ein dichtgepacktes Kristallbett bildet. Das Kristallbett wird von der Mutterlauge in Richtung des Bodens der Kolonne durchströmt und durch den Strömungswiderstand nach unten gedrückt. Am Boden der Kolonne befindet sich eine bewegte, vorzugsweise rotierende Kratzvorrichtung oder Kratzer, die aus dem dichtgepackten Kristallbett und der am unteren Teil der Waschkolonne eingebrachten Waschschmelze wieder eine Suspension erzeugt. Diese Suspension wird vorzugsweise durch einen Aufschmelzer, vorzugsweise einen Wärmetauscher, gepumpt und aufgeschmolzen. Ein Teil der Schmelze kann z.B. als Waschschmelze dienen; diese wird dann in die Kolonne zurückgepumpt und wäscht vorzugsweise das in entgegengesetzter Richtung wandernde Kristallbett aus, d.h. die kristallisierte (Meth)Acrylsäure wird im Gegenstrom von der zurückgeführten (Meth)Acrylsäure gewaschen. Die Waschschmelze bewirkt einerseits ein Waschen der Kristalle, andererseits kristallisiert die Schmelze auf den Kristallen zumindest teilweise aus. Die freiwerdende Kristallisationsenthalphie erwärmt das Kristallbett im Waschbereich der Kolonne. Dadurch wird ein dem Schwitzen der Kristalle analoger Reinigungseffekt erzielt. Damit wird eine Aufreinigung zum einen durch das Waschen der Oberfläche der (Meth)Acrylsäurekristalle mit aufgeschmolzener - und damit bereits gereinigter-(Meth)Acrylsäure bewirkt, zum anderen wird durch die Kristallisation der aufgeschmolzenen, gereinigten (Meth)Acrylsäure auf den bereits vorhandenen (Meth)Acrylsäurekristallen ein Ausheilen bzw. Ausschwitzen von Verunreinigungen erreicht. Dieses erlaubt die hochreine Herstellung der (Meth)Acrylsäure.

In einer speziellen Ausgestaltung des erfindungsgemäßen Verfahrens ist der Rückführstrom, der sich aus der Zurückführung der aufgeschmolzenen (Meth)Acrylsäure aus dem Schritt c) in den Schritt a) bzw. in den Schritt b) ergibt, größer als ein Feedstrom der Zusammensetzung, der von außen kontinuierlich dem Schritt a) zugeführt wird.

Insbesondere ist der Rückführstrom mindestens doppelt, vorzugsweise mindestens zehnmal so groß wie der Feedstrom. Durch den großen Rückführstrom wird sichergestellt, dass die thermische Belastung der (Meth)Acrylsäure am Aufschmelzer verringert wird.

Zur Impfung der zu kristallisierenden (Meth)Acrylsäure ist es vorteilhaft die abgetrennte, kristallisierte (Meth)Acrylsäure aus Schritt b) zumindest teilweise in den Schritt a) zuführen. Die zurückgeführten (Meth)Acrylsäure-kristalle erleichtern das Kristallwachstum im Schritt a) und unterstützen so die Trennung der (Meth)Acrylsäure von der Mutterlauge. Dieses ist insbesondere bei einer wässrigen Zusammensetzung vorteilhaft.

Grundsätzlich ist aufgrund energetischer Überlegungen ein einstufiges Aufreinigungsverfahren mit nur einer Verfahrensstufe besonders vorteilhaft und damit besonders bevorzugt. Unter Umständen ist jedoch ein zweistufiges Aufreinigungsverfahren sinnvoll.

Zur Steigerung der Ausbeute ist es zweckmäßig, die nach Schritt b) abgetrennte Mutterlauge zumindest teilweise in den Schritt a) zurückzuführen. In der Mutterlauge verbliebene (Meth)Acrylsäure kann so mit geeigneten räumlichen Temperaturprofilen, vorzugsweise bis zum thermodynamischen Limit (z.B. Eutektikum) weiter auskristallisiert werden.

In einer vorteilhaften Gruppe von Ausgestaltungen weist das erfindungsgemäße Verfahren mindestens zwei Verfahrensstufen auf, die jeweils die Schritte a) bis d) aufweisen, wobei mindestens eines der folgenden Merkmale (α1) bis (α4) erfüllt ist:
(α1) abgetrennte, insbesondere kristalline und/oder aufgeschmolzene, (Meth)Acrylsäure aus einer ersten Verfahrensstufe wird zumindest teilweise einer zweiten Verfahrensstufe zugeführt;
(α2) abgetrennte, insbesondere kristalline und/oder aufgeschmolzene, (Meth)Acrylsäure aus einer zweiten Verfahrensstufe wird zumindest teilweise einer ersten Verfahrensstufe zugeführt;
(α3) Mutterlauge, insbesondere nach dem Schritt b) abgetrennte Mutterlauge, einer ersten Verfahrensstufe wird zumindest teilweise einer zweiten Verfahrensstufe zugeführt;
(α4) Mutterlauge, insbesondere nach dem Schritt b) abgetrennte Mutterlauge, einer zweiten Verfahrensstufe wird zumindest teilweise einer ersten Verfahrensstufe zugeführt.

Aus dieser Gruppe vorteilhafter Ausgestaltungen mit mindestens einem der Merkmale (α1) bis (α4) der Erfindung sind die Ausgestaltungen bevorzugt, bei den mindestens eines der folgenden Merkmale (β1) und (β6) erfüllt ist:
(β1) kristalline (Meth)Acrylsäure aus der ersten Verfahrenstufe wird zumindest einem der Schritte a) und b) der zweiten Verfahrenstufe zugeführt;
(β2) aufgeschmolzene (Meth)Acrylsäure aus der ersten Verfahrenstufe wird zumindest einem der Schritte a) und b) der zweiten Verfahrenstufe zugeführt;
(β3) kristalline (Meth)Acrylsäure aus der zweiten Verfahrensstufe wird zumindest einem der Schritte a), b) und c) der ersten Verfahrensstufe zugeführt;
(β4) aufgeschmolzene (Meth)Acrylsäure aus der zweiten Verfahrenstufe wird zumindest einem der Schritte a), b) und c) der ersten Verfahrenstufe zugeführt;
(β5) die nach dem Schritt b) abgetrennte Mutterlauge der ersten Verfahrenstufe wird zumindest teilweise dem Schritt a) der zweiten Verfahrenstufe zugeführt;
(β6) die nach dem Schritt b) abgetrennte Mutterlauge der zweiten Verfahrenstufe wird zumindest teilweise dem Schritt a) der ersten Verfahrenstufe zugeführt.

Aus dieser Gruppe bevorzugter Ausführungsformen der Erfindung sind für den Fall, dass die zweite bzw. weitere Verfahrensstufe eine zusätzliche Aufreinigung der (Meth)Acrylsäure bewirkt, d.h. bereits in einer ersten Verfahrensstufe gereinigte (Meth)Acrylsäure in einer zweiten (weiteren) Verfahrensstufe weiter gereinigt wird, folgende Ausführungsformen besonders bevorzugt:
(1) zur Erzielung einer guten Reinheit der (Meth)Acrylsäure wird aufgeschmolzene (Meth)Acrylsäure aus der ersten Verfahrensstufe dem Schritt a) der zweiten Verfahrensstufe zugeführt;
(2) zur Erzielung einer guten Reinheit der (Meth)Acrylsäure wird kristalline (Meth)Acrylsäure aus der ersten Verfahrensstufe dem Schritt a) der zweiten Verfahrensstufe zugeführt;
(3) um gründliches Waschen der (Meth)Acrylsäurekristalle der ersten Stufe zu ermöglichen, wird aufgeschmolzene (Meth)Acrylsäure aus der zweiten Verfahrensstufe dem Schritt b) der ersten Verfahrensstufe zugeführt; oder
(4) um besonders reine Impfkristalle zur Verfügung zu stellen wird kristalline (Meth)Acrylsäure aus der zweiten Verfahrensstufe dem Schritt a) der ersten Verfahrensstufe zugeführt.

Hierbei ganz besonders bevorzugt ist eine Kombination aus der Ausführungsformen (1) bzw. (2) und (3).

Aus der Gruppe bevorzugter Ausführungsformen der Erfindung sind für den Fall, dass die zweite Verfahrensstufe zur Steigerung der Ausbeute dient, folgende Ausführungsformen besonders bevorzugt:
(1) kristalline (Meth)Acrylsäure aus der ersten Verfahrensstufe werden als Impfkristalle zur Kristallisation dem Schritt a) der zweiten Verfahrensstufe zugeführt;
(2) zur Steigerung der Ausbeute wird die nach Schritt b) abgetrennte Mutterlauge der ersten Verfahrensstufe zumindest teilweise dem Schritt a) der zweiten Verfahrensstufe zugeführt;
(3) zur Steigerung der Reinheit wird aufgeschmolzene (Meth)Acrylsäure aus dem zweiten Verfahrensschritt dem Schritt a) der ersten Verfahrensstufe zugeführt; oder
(4) zur Minimierung des Energieaufwands wird kristalline (Meth)Acrylsäure aus dem zweiten Verfahrensschritt dem Schritt a) der ersten Verfahrensstufe zugeführt.

Hierbei ganz besonders bevorzugt ist eine Kombination aus den Ausführungsformen (2) und (4).

Vorteilhafterweise sind mindestens zwei Verfahrensstufen in Reihe vorgesehen. In Reihe kann sich sowohl auf die abgetrennte (Meth)Acrylsäure, d.h. auf die kristalline oder auf die aufgeschmolzene (Meth)Acrylsäure, als auch auf die abgetrennte Mutterlauge beziehen.

Vorteilhaft ist es auch mindestens zwei Verfahrenstufen miteinander zu verschachteln. Hierdurch wird bewirkt, dass die Anzahl der für das Aufschmelzen notwendigen Aufschmelzer, die als Wärmetauscher bzw. Heizungen energieaufwendig im Betrieb sind, geringer ist als die Anzahl der Stufen. Beispielsweise kann eine einfache Verschachtelung von zwei Verfahrensstufen eine Ersparnis eines Aufschmelzers bedeuten. Hiermit wird der Betrieb bei gleicher Ausbeute und gleicher Reinheit deutlich kostengünstiger.

Die Anzahl der Verfahrensstufen richtet sich nach der mit dem Verfahren zu erzielenden Reinheit und Wirtschaftlichkeit. Grundsätzlich ist die erzielbare Reinheit von (Meth)Acrylsäure durch das thermodynamische Limit (z.B. Eutektikum) für eine Kristallisierbarkeit von (Meth)Acrylsäure aus der Mutterlauge begrenzt.

Eine spezielle Ausgestaltung des erfindungsgemäßen Verfahren ist gekennzeichnet durch folgende Merkmale (γ1) und (γ2):
(γ1) Kristallisation von (Meth)Acrylsäure aus einem verunreinigten Roh-(Meth)Acrylsäurestrom aus einem Verfahren zur Herstellung von (Meth)Acrylsäure;
(γ2) Abtrennen der (Meth)Acrylsäurekristalle aus der Mutterlauge mittels einer Waschkolonne, wobei man die Mutterlauge aus Schritt (γ2) zumindest teilweise in den Schritt (γ1) zurückführt, wobei der Roh-(Meth)Acrylsäurestrom eine Reinheit von <99,5 Gew.-% an (Meth)Acrylsäure aufweist.

Die erfindungsgemäße Vorrichtung zur Herstellung von (Meth)Acrylsäure beinhaltet als fluidleitend miteinander verbundene Komponenten eine (Meth)Acrylsäure-Syntheseeinheit, die vorzugsweise einen (Meth)Acrylsäure-Reaktor, einen Quenchabsorber beinhaltet, eine Destillationsvorrichtung und eine Aufreinigungsvorrichtung, wobei die Aufreinigungsvorrichtung eine Vorrichtungseinheit aufweist, welche die Merkmale (δ1) bis (δ4) umfasst:
(δ1) die Vorrichtungseinheit weist einen Kristallisationsbereich, einen Trennbereich, einen Aufschmelzer, und mindestens drei Führungen auf;
(δ2) der Kristallisationsbereich ist mit dem Trennbereich über eine erste Führung verbunden;
(δ3) der Trennbereich ist mit dem Aufschmelzer über eine zweite Führung verbunden;
(δ4) der Aufschmelzer ist mit dem Kristallisationsbereich über eine dritte Führung oder mit dem Trennbereich über eine vierte Führung verbunden;
wobei die Aufreinigungsvorrichtung einen Zulauf aufweist, der eine Zusammensetzung, beinhaltend (Meth)Acrylsäure, mindestens eine Verunreinigung und Wasser, wobei die Zusammensetzung einen Wassergehalt im Bereich von 0,55 bis 90, vorzugsweise von 7 bis 50 und besonders bevorzugt von 10 bis 25 Gew.-%, bezogen auf die Zusammensetzung, aufweist, führt.

Unter "fluidleitend" wird erfindungsgemäß verstanden, dass Gase oder Flüssigkeiten, Suspensioneneingeschlossen, oder deren Mischungen durch entsprechende Leitungen geführt werden. Hierzu lassen sich insbesondere Rohleitungen, Pumpen und der gleichen einsetzen.

Aufgrund des geringen Destillationsgrades wird die so hergestellte (Meth)Acrylsäure besonders schonend behandelt, wodurch ihre Qualität verbessert wird. Außerdem kann die so gewonnen reine wässrige (Meth)Acrylsäure in Lösungs-, Emulsions- oder Suspensionspolymerisationen eingesetzt werden, wodurch gegenüber der herkömmlichen Reaktionsführung ein nachteiliger Lösungsmittelwechsel von gleichen oder unterschiedlichen Lösungsmitteln vermieden wird.

In einer Ausführungsform der erfindungsgemäßen Vorrichtung ist es bevorzugt, dass die (Meth)Acrylsäure-Syntheseeinheit und die Aufreinigungsvorrichtung ohne eine Destillationsvorrichtung miteinander verbunden sind.

In einer anderen Ausführungsform der erfindungsgemäßen Vorrichtung ist es bevorzugt, dass der Quenchabsorber und die Aufreinigungsvorrichtung ohne eine Destillationsvorrichtung miteinander verbunden sind.

Eine erfindungsgemäß bevorzugte Vorrichtung zur Herstellung von Acrylsäure weist in dem Bereich, der einen (Meth)Acrylsäurereaktor und einen Quenchabsorber aufweist, vorzugsweise folgenden Aufbau bei der Synthese von Acrylsäure auf: Propylen und ggf. weitere Inertgase wie Stickstoff oder Verbrennungsgase wie CO₂ oder Stickoxide werden in einen ersten Reaktor zu einer ersten katalytischen Oxidation über eine Eduktzufuhr, die in den ersten Reaktor mündet zugeleitet. Der erste Reaktor ist über eine weitere Leitung mit einem zweiten Reaktor verbunden, in den das Produkt der ersten katalytischen Oxidation aus dem ersten Reaktor für eine zweite katalytische Oxidation eingeleitet wird. Das Acrylsäure beinhaltende Produkt der zweiten katalytischen Oxidation wird über eine zwischen dem zweiten Reaktor und dem Quenchabsorber befindlichen Leitung der unteren Hälfte des Quenchabsorbers zugeführt. In dem Quenchabsorber wird das Produkt der zweiten katalytischen Oxidation mit Wasser in Kontakt gebracht, wobei das Wasser oberhalb der Zuführung des Produkts der zweiten katalytischen Oxidation in den Quenchabsorber eingespeist wird. Zum einen wird eine Acrylsäure und Wasser beinhaltende erste Phase unterhalb der Zuführung des Produkts der zweiten katalytischen Oxidation aus dem Quenchabsorber abgeführt. Diese erste Phase kann zumindest teilweise wieder oberhalb der Zuführung des Produkts der zweiten katalytischen Oxidation wieder in den Quenchabsorber zurückgeführt werden. Die nicht in den Quenchabsorber zurückgeführte erste Phase wird der Destillationsvorrichtung zugeführt, um beispielsweise einer azeotropen Trennung unterzogen zu werden, in der die Acrylsäure aufkonzentriert und gereinigt wird. Oberhalb der Rückführung der ersten Phase und unterhalb der Einspeisung von Wasser in den Quenchabsorber kann eine Acrylsäure und Wasser beinhaltende zweite Phase aus dem Quenchabsorber abgeführt werden. Diese zweite Phase kann genauso wie die erste Phase der Destillationsvorrichtung zugeführt werden, um beispielsweise einer azeotropen Trennung unterzogen zu werden, in der die Acrylsäure aufkonzentriert und gereinigt wird. Die aus dem Quenchabsorber abgeleiten Abgase können einer katalytischen Verbrennung zugeführt werden. Die Verbrennungsgase der katalytischen Verbrennung können als Inertgase in den ersten Reaktor eingespeist werden. Das bei der Aufkonzentration von Acrylsäure wiedergewonnene Wasser kann in den Quenchabsorber zurückgeführt werden. Weitere Einzelheiten zur Herstellung von Acrylsäure sind in DE 197 40 252 A1 offenbart, auf deren Inhalt hiermit als Teil dieser Offenbarung Bezug genommen wird.

Die Synthese von Acrylsäure kann erfindungsgemäß auch in einer Syntheseeinheit erfolgen, die Propan direkt zu Acrylsäure umsetzt. Außerdem kann die Synthese von Acrylsäure in wässrigem Reaktionsmedien, bevorzugt als homogene Katalyse, erfolgen. Bei einer Reaktion in wässerigen Reaktionsmedien würde die Acrylsäure in Form der wasserreichen Zusammensetzung anfallen, ohne dass eine Wasserzugabe in einem Quenchabsorber notwendig wäre.

Eine erfindungsgemäß bevorzugte Vorrichtung zur Herstellung von Methacrylsäure weist den Methacrylsäurereaktor und einen Quenchabsorber bei der Synthese von Methacrylsäure durch katalytische Gasphasenreaktion von C4-Ausgangsverbindungen mit Sauerstoff auf. Besonders bevorzugt ist Methacrylsäure durch katalytische Gasphasenoxidation von Isobuten, Isobutan, tert.-Butanol, iso-Butyraldehyd, Methacrolein oder Meth-tert.-butylether erhältlich. Weitere Einzelheiten zur Herstellung von (Meth)Acrylsäure sind in EP 0 092 097 B1, EP 0 058 927 und EP 0 0608 838 offenbart, auf deren Inhalt hiermit als Teil dieser Offenbarung Bezug genommen wird.

Die Aufreinigungsvorrichtung ist in der Lage aus einem vergleichsweise verunreinigten Roh-(Meth)Acrylsäurestrom von rund 85 Gew.-% sehr reine (Meth)Acrylsäure mit Reinheitsgraden von über 99,5 Gew.-% zu gewinnen. Es ist nach der Erfindung möglich, verunreinigte Roh- (Meth)Acrylsäurestrom mit 50 Gew.-% bis 95 Gew.-% (Meth)Acrylsäure, vorzugsweise 75 Gew.-% bis 90 Gew.-% (Meth)Acrylsäure effizient aufzureinigen. Diese effektive Aufreinigung erlaubt, die Vorreinigung des Roh-(Meth)Acrylsäurestroms mit Hilfe der Destillationsvorrichtung zu reduzieren, wodurch die thermische Belastung der (Meth)Acrylsäure reduziert wird. Hiermit wird die Qualität der (Meth)Acrylsäure verbessert.

Zur weiteren Steigerung der Reinheit der (Meth)Acrylsäure weist die Vorrichtungseinheit eine separate Reinigungsvorrichtung auf. Diese separate Reinigungsvorrichtung kann zur weiteren Aufreinigung des Endprodukts, insbesondere zur weiteren Aufreinigung der den Aufschmelzer verlassenden (Meth)Acrylsäure, eingesetzt werden.

Zur verbesserten Kristallisation ist vorzugsweise der Trennbereich mit dem Kristallisationsbereich durch eine erste Rückführung für abgetrennte (Meth)Acrylsäure verbunden.

Zur Steigerung der Ausbeute ist vorteilhafterweise der Trennbereich mit dem Kristallisationsbereich durch eine zweite Rückführung für abgetrennte Mutterlauge verbunden ist.

Grundsätzlich ist aufgrund energetischer Überlegungen eine einstufige Aufreinigungsvorrichtung mit nur einer Vorrichtungseinheit besonders vorteilhaft und damit besonders bevorzugt. Unter Umständen ist jedoch eine zweistufige Aufreinigungsvorrichtung sinnvoll.

Zur Steigerung der Effektivität der Aufreinigung, insbesondere zur Steigerung der Reinheit und der Ausbeute, enthält die erfindungsgemäße Vorrichtung mindestens zwei Vorrichtungseinheiten gemäß den Merkmalen (δ1) bis (δ4), die durch mindestens eine Verbindungsleitung verbunden sind, wobei die Verbindungsleitung eine Zuleitung oder eine Rückleitung ist, und wobei mindestens eines der folgenden Merkmale (ε1) bis (ε4) erfüllt ist:
(ε1) der Trennbereich einer ersten Vorrichtungseinheit ist über die Verbindungsleitung mit dem Kristallisationsbereich einer zweiten Vorrichtungseinheit verbunden;
(ε2) der Aufschmelzer einer ersten Vorrichtungseinheit ist über die Verbindungsleitung mit dem Kristallisationsbereich einer zweiten Vorrichtungseinheit verbunden;
(ε3) der Trennbereich einer zweiten Vorrichtungseinheit ist über die Verbindungsleitung mit dem Kristallisationsbereich einer ersten Vorrichtungseinheit verbunden;
(ε4) der Aufschmelzer einer zweiten Vorrichtungseinheit ist über die Verbindungsleitung mit dem Kristallisationsbereich einer ersten Vorrichtungseinheit verbunden. Hierbei können die Verbindungsleitungen sowohl Zu- wie auch Rückleitung sein.

Beispielsweise ist es zweckmäßig entsprechend des erfindungsgemäßen Verfahrens zur Steigerung der Ausbeute von dem Trennbereich der ersten Vorrichtungseinheit (erste Verfahrensstufe) eine Zuleitung zum Kristallisationsbereich der zweiten Vorrichtungseinheit (zweite Verfahrensstufe) vorzusehen. Eine Rückführung zwischen dem Trennbereich der zweiten Stufe und dem Kristallisationsbereich der ersten Stufe ist hingegen zur Bereitstellung von Impfkristallen zweckmäßig.

Damit ist es auf vorteilhafte Weise möglich, zur Verbesserung der Reinheit und der Ausbeute eine Reihenschaltung von mindestens zwei Vorrichtungseinheiten vorzusehen.

Durch eine Verschachtelung von mindestens zwei Vorrichtungseinheiten kann die Anzahl der erforderlichen Aufschmelzer verringert und damit der Energieaufwand zum Betrieb der Vorrichtung reduziert werden.

Bei dem erfindungsgemäßen Verfahren zur Aufreinigung von Acrylsäure herrschen in dem Trennbereich eine Temperatur im Bereich von -20 bis 20°C, vorzugsweise von - 10 bis 13°C bei einem Druck von 1 bis 10 bar. Es ist bevorzugt, dass in dem unteren Bereich des Trennbereichs eine geringere Temperatur und ein geringerer Druck herrschen, als im oberen Bereich des Trennbereichs. Vorzugsweise herrschen in dem unteren Bereich des Trennbereichs -20 bis < 12 °C bei einem Druck von 1 bis 2 bar. Im oberen Bereich des Trennbereichs herrscht eine Temperatur von mindestens 12°C und ein Druck von 1 bis 10 bar, vorzugsweise 3 bis 7 bar.

Bei dem erfindungsgemäßen Verfahren zur Aufreinigung von Acrylsäure herrschen in dem Kristallisationsbereich eine Temperatur im Bereich von -20 bis 20°C, vorzugsweise von -12 bis 13°C bei einem Druck von 0,5 bis 10 bar, vorzugsweise 0,8 bis 2 bar.

Bei dem erfindungsgemäßen Verfahren zur Aufreinigung von Acrylsäure herrschen in dem Aufschmelzer eine Temperatur im Bereich von 10 bis 50°C, vorzugsweise von 11 bis 30°C bei einem Druck von 1 bis 10 bar, vorzugsweise 3 bis 7 bar.

Bei dem erfindungsgemäßen Verfahren zur Aufreinigung von Methacrylsäure herrschen in dem Trennbereich eine Temperatur im Bereich von -5 bis 30°C, vorzugsweise von -3 bis 20°C bei einem Druck von 1 bis 10 bar. Es ist bevorzugt, dass in dem unteren Bereich des Trennbereichs eine geringere Temperatur und ein geringerer Druck herrschen, als im oberen Bereich des Trennbereichs. Vorzugsweise herrschen in dem unteren Bereich des Trennbereichs -16 bis < 15°C bei einem Druck von 1 bis 2 bar. Im oberen Bereich des Trennbereichs herrscht eine Temperatur von mindestens 15°C und ein Druck von 1 bis 10 bar.

Bei der erfindungsgemäßen Vorrichtung zur Aufreinigung von Methacrylsäure herrschen in dem Kristallisationsbereich eine Temperatur im Bereich von -5 bis 30°C, vorzugsweise von -3 bis 20°C bei einem Druck von 1 bis 10 bar, vorzugsweise 1 bis 2 bar.

Bei der erfindungsgemäßen Vorrichtung zur Aufreinigung von Methacrylsäure herrschen in dem Aufschmelzer eine Temperatur im Bereich von 10 bis 50°C, vorzugsweise von 11 bis 30°C bei einem Druck von 1 bis 10 bar, vorzugsweise 3 bis 7 bar.

In den Führungen herrschen Temperatur- und Druckverhältnisse, die einen sicheren und störungsfreien Transport der (Meth)Acrylsäure und den diese ggf. begleitenden Stoffe in diesen Führungen erlauben.

Die erfindungsgemäße Vorrichtung erlaubt es, von einer verhältnismäßig verunreinigten (Meth)Acrylsäure auszugehen, wodurch der Voraufwand für eine Destillation der aus der Synthese stammenden (Meth)Acrylsäure geringer wird. Damit sinkt insbesondere die thermische Belastung der (Meth)Acrylsäure, die zu unerwünschten Polymerisationen führen kann.

Weiterhin betrifft die Erfindung eine Vorrichtung zur Polymerisation von (Meth)Acrylsäure, beinhaltend eine Vorrichtung zur Herstellung von (Meth)Acrylsäure und eine Polymerisationseinheit, wobei die Aufreinigunsvorrichtung der Vorrichtung zur Herstellung von (Meth)Acrylsäure mit der Polymerisationseinheit verbunden sind.

Die Polymerisation erfolgt vorzugsweise als Lösungspolymerisation, wobei die Reaktionsführung in einem Muldenband besonders bevorzugt ist. Hierbei wird entweder die wässrige Phase unmittelbar eingesetzt oder die wasserarme Phase entsprechend verdünnt. Im allgemeinen erfolgt die Polymerisation in einem Medium mit einem Wassergehalt von 20 bis 80 Vol.-%, bezogen auf das Medium.

Bei dem nach dem erfindungsgemäßen Verfahren erhältlichen Polymer handelt es sich vorzugsweise um ein Absorbierendes Polymer mit einer maximale Aufnahme von 0.9 Gew.-%iger wässriger NaCl-Lösung gemäß ERT 440.1-99 in einem Bereich von 10 bis 1000, bevorzugt von 15 bis 500 und besonders bevorzugt von 20 von 300 ml/g. Weitere Einzelheiten zu absorbierenden Polymeren und deren Herstellung ergeben sich aus *"*Modern Superabsorbent Polymer Technology" Fredric L. Buchholz, Andrew T. Graham, Whiley-VCH, 1998.

Die nach dem erfindungsgemäßen Verfahren erhältliche (Meth)Acrylsäure oder das nach dem erfindungsgemäße Verfahren erhältliche Polymer wird in oder zur Herstellung von Fasern, Formkörpern, Filmen, Schäumen, superabsorbierenden Polymeren oder Hygieneartikeln verwendet.

Die erfindungsgemäße Vorrichtung oder die erfindungsgemäße Aufreinigungsvorrichtung oder das erfindungsgemäßen Verfahren wird zur Herstellung von Acrylsäure verwendet, die eine Reinheit von mehr als 90 Gew.-%, bevorzugt mehr als 95 Gew.-% und besonders bevorzugt mehr als 99,5 Gew.-%, jeweils bezogen auf die (Meth)Acrylsäure mit Verunreinigungen, aufweist.

In einer speziellen Ausgestaltung des erfindungsgemäßen Verfahren zur Aufreinigung von (Meth)Acrylsäure weist das Verfahren zwei Verfahrensschritte auf:
γ1) Kristallisation von (Meth)Acrylsäure aus einem verunreinigten Roh-(Meth)Acrylsäurestrom aus einem Verfahren zur Herstellung von (Meth)Acrylsäure;
γ2) Abtrennen der (Meth)Acrylsäurekristalle aus der Mutterlauge mittels einer Waschkolonne, die Mutterlauge aus Schritt γ2 zumindest teilweise in den Schritt γ1) zurückführt, wobei der Roh-(Meth)Acrylsäurestrom vorzugsweise eine Reinheit von <99,5 Gew.-% an (Meth)Acrylsäure aufweist,

Eine mechanisch betriebene Waschkolonne (Diss. Poschmann, S.18) ist erfindungsgemäß besonders bevorzugt.

Bei einer mechanischen Waschkolonne - beispielhaft wird auf die EP 0 193 226 B und NL 1007687 A verwiesen - wird ein dichtes Kristallbett innerhalb der Kolonne mittels eines für die Schmelze durchlässigen Kolbens erzeugt. Der Kolben kann sich am oberen oder unteren Ende der Kolonne befinden; im ersten Fall erfolgt der Zulauf der Suspension im oberen Bereich der Kolonne, im zweiten Fall im mittleren oder unteren Bereich. Der Kolben ist für die Schmelze durchlässig, so dass beim Komprimieren Schmelze an der Rückseite des Kolbens austritt und dort abgezogen wird. Analog wie eine hydraulische Waschkolonne enthält auch die mechanische Waschkolonne eine Vorrichtung zum Abkratzen, beispielsweise ein bewegtes, vorzugsweise rotierendes Kratzorgan, um Kristalle aus dem Kristallbett abzukratzen und mit der Waschschmelze in eine Suspension zu überführen. Die Waschschmelze bewegt sich im Gegenstrom zum Kristallbett. Von der dem Kolben gegenüberliegenden Seite der Waschkolonne wird Suspension abgezogen, und nach dem Aufschmelzen kann ein Teil der Schmelze als Waschschmelze zurückgeführt und der andere Teil als Reinstprodukt aus dem Kreislauf abgezogen werden.

Ausführungsformen zur Suspensionskristallisation mit nachgeschalteter Wäsche der Kristalle in einer hydraulischen oder mechanischen Waschkolonne sind dem Buch "Melt Crystallization Technology" von G.F. Arkenbout, Technomic Publishing Co. Inc., Lancaster-Basel (1995), S. 265-288 sowie dem sich auf die Niro-Gefrierkonzentration zur Abwasservorkonzentrierung richtenden Artikel in Chemie Ingenieur Technik (72) (10/2000), 1231-1233 zu entnehmen.

Als Waschflüssigkeit kann je nach Einsatzzweck eine dem Fachmann geläufige Waschflüssigkeit benutzt werden (bei wässrigen Lösungen z.B. Wasser). Ganz besonders bevorzugt dienen zum Waschen der kristallisierten (Meth)Acrylsäure wie schon angedeutet eine Teilmenge der aufgeschmolzenen Kristalle derselben. Durch diese Maßnahme wird zum einen gewährleistet, dass zur Produktion hochreiner Produkte kein weiterer Stoff in das System eingeführt werden muß, zum anderen dienen die aufgeschmolzenen Kristalle aber auch zum Zurückdrängen der Mutterlaugenfront in der Waschkolonne und üben gleichzeitig auf die Kristalle einen reinigenden Effekt analog dem Schwitzen aus. Ein Produktverlust findet dabei nicht statt, da die Waschflüssigkeit auf den zu waschenden Kristallen wiederum auskristallisiert und sich so im Produkt wiederfindet (Z. B. Prospekt der Firma Niro Process Technology B.V., Crystallization and wash column separation set new standards in purity of chemical compounds).

Weiterhin ist es in dem erfindungsgemäßen Verfahren bevorzugt, dass das Verfahren in einer erfindungsgemäßen Vorrichtung erfolgt.

In einer Ausgestaltung des erfindungsgemäßen Verfahrens wird die abgetrennte, insbesondere kristalline und/oder geschmolzene, (Meth)Acrylsäure in einem separaten Reinigungsverfahren gereinigt. Beispielsweise ist es möglich, die Mutterlauge aus Schritt b) vor dem Zurückführen in den Schritt a) mindestens ein Mal mit einem weiteren Reinigungsverfahren zu behandeln. Derartige Verfahren sind dem Fachmann hinlänglich bekannt. Vorzugsweise kommen als solche Verfahren im einzelnen folgende zur Anwendung:

### 1.) Einfache Destillation

Auftrennung in Leichtsieder (Essigsäure, Wasser usw.), Mittelsieder (Meth(Acrylsäure)) und Schwersieder (MSA, PTA usw.). Die Reinigung verunreinigter (Meth)Acrylsäure (insbes. von Wasser und Essigsäure) wird in einem Großteil der Fälle mittels Azeotroprektifikation vorgenommen. Zur Anwendung kommen beispielsweise Schleppmittel wie Toluol oder MIBK (EP 0 695 736 B1).

### 2.) Extraktion von Acrylsäure

Mit n-Butanol kann die (Meth)Acrylsäure extraktiv gewonnen werden. Übrig bleibt eine wässrige Phase, in der die Nebenkomponenten gelöst sind. Die Extraktion von (Meth)Acrylsäure aus verunreinigten Lösungen ist Stand der Technik wie die Destillation.
Extrahiert werden kann (Meth)Acrylsäure beispielsweise auch aus wässrigen Lösungen mit flüssigen Ionenaustauschern, Mischungen aus tri-n-Alkylaminen und aliphatischen Alkoholen oder n-Butanol (Vogel et al.: Chem.Eng.Technol.23 (2000)1, pp. 70 - 74; Tamada et al.: in Solvent Extraction 1990, Ed.: T. Sekine, Elsevier Science Publishers B.V., pp. 1791 - 1796; JP 57 095 938; WO 98/40342; Informationsbroschüre der Firma SULZER Chemtech zur fraktionierten Extraktion von (METH)ACRYLSÄURE mit n-Butanol).

### 3.) Entwässerung von (Meth)Acrylsäure durch Pervaporation

Dieses Verfahren ist unter anderem in DE 4401405 A1 offenbart.

Vorteilhafterweise kann man die Mutterlauge aus Schritt b) vor dem Zurückführen in den Schritt a) mindestens ein Mal mit einem Verfahren aufweisend die Schritte a) und b) behandeln und den jeweils reineren abgezweigten Teilstrom in Schritt a) des Ausgangs- oder eines Vorgängerverfahrens zurückführen, um dadurch mit minimalisiertem Ausbeuteverlust ein Maximum an Reinheit zu erzeugen.

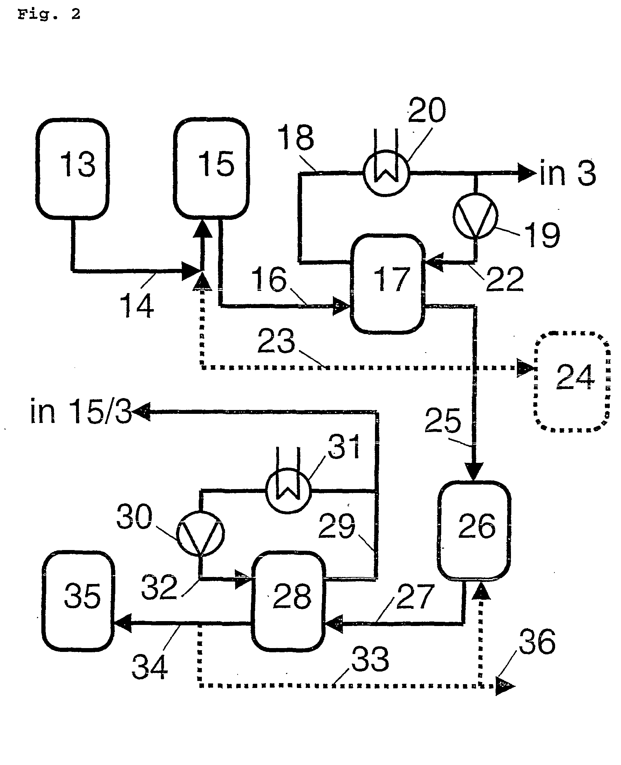

Fig. 2 erläutert diesen Sachverhalt. Die Mutterlauge der Kristalltrennung in der Waschkolonne kann ebenfalls in einem nächsten Kristallisationsbereich behandelt werden. Die entstehende Suspension kann dann wiederum in einer Waschkolonne wie gehabt aufgearbeitet werden. Die jetzt entstandene Mutterlauge kann in folgenden Stufen analog behandelt werden. Man hat dabei die Wahl, die bei dieser Verfahrensweise anfallenden jeweils reineren Teilströme in die Kristallisation des ersten Reinigungsverfahrens zurückzuführen oder aber in die Kristallisation eines Vorgängerverfahrens. Derart kann mit relativ geringem apparativen Aufwand ein Maximum an Reinheit bei einem Minimum an Abfall realisiert werden.

Die erfindungsgemäßen Verfahren können kontinuierlich und diskontinuierlich betrieben werden, wobei der kontinuierliche Betrieb bevorzugt ist, da ein solcher Betrieb besonders wirtschaftlich ist.

Die Erfindung betrifft weiterhin eine Vorrichtung zur Polymerisation von (Meth)Acrylsäure, beinhaltend eine erfindungsgemäße Vorrichtung zur Herstellung von (Meth)Acrylsäure und eine Polymerisationseinheit, wobei die Aufreinigunsvorrichtung der Vorrichtung zur Herstellung von (Meth)Acrylsäure mit der Polymerisationseinheit verbunden ist. Bei dieser Polymerisationseinheit kann es sich um einen Knet- oder Rührreaktor oder ein Muldenband handeln in bzw. auf dem die Polymerisation erfolgt. Bei der wasserreichen Phase ist die Muldenband Polymerisation vorteilhaft. Die wasserreiche Phase weist eine Wasser- und (Meth)Acrylsäurekonzentration auf, die sich besonders für die Lösungspolymerisation von auf (Meth)Acrylsäure basierenden Polymeren, vorzugsweise superabsorbierende Polymere und Spezialpolymere für die Leder- und Papierherstellung sowie die Abwasserbehandlung, eignet.

Die aus dem erfindungsgemäßen Verfahren erhältlichen Polymere sind vorzugsweise Superabsorber, Spezialpolymere für die Bereiche Abwasserbehandlung, Dispersionsfarben, Kosmetika, Textilien, Lederveredelung oder Papierherstellung.

Unter superabsorbierenden Polymeren werden Polymere verstanden, die ein mehrfaches ihres Eigengewicht an Wasser oder wässrigen Flüssigkeiten aufnehmen. Vorzugsweise basieren superabsorbierende Polymere zu mehr als der Hälfte auf Acrylssäure als Monomer. Weitere Einzelheiten zu superabsorbierenden Polymeren, ihrer Herstellung und Verwendung in Hygieneartikeln sind "Modern Superabsorbent Polymer Technology", Fredric L. Buchholz, Andrew T. Graham, Wiley-VCH, 1998 zu entnehmen, auf dessen Inhalt als Teil dieser Offenbarung Bezug genommen wird. Diese superabsorbierenden Polymere werden bevorzug in Hygieneartikel eingearbeitet. Unter den Hygieneartikeln sind Windeln, Erwachsenen Inkontinenzartikel und Damenbinden besonders bevorzugt.

Weiterhin stellen die sich aus den vorstehend mit Ziffern gekennzeichneten Merkmalen ergebenden Kombinationen von Merkmalen jeweils einzelne Ausführungsformen der vorliegenden Erfindung dar.

Weitere Einzelheiten und vorteilhafte Weiterbildungen werden anhand der folgenden Zeichnung, die die Erfindung exemplarisch veranschaulichen soll, näher erläutert.

Es zeigen schematisch:
- Fig. 1: ein weiteres erfindungsgemäßes Verfahrensschema nach Fig. 1;
- Fig. 2: ein erfindungsgemäßes Verfahrensschema mit zwei Verfahrensstufen in einer Reihenschaltung;
- Fig. 3: ein erfindungsgemäßes Verfahrensschema mit zwei Verfahrensstufen in Verschachtelung;
- Fig. 4: ein erfindungsgemäßes Verfahrensschema mit einer Verfahrensstufe;
- Fig. 5: ein erfindungsgemäßes Verfahrensschema mit zwei Verfahrensstufen;
- Fig. 6: einen Kristallhabitus reiner Acrylsäure;
- Fig. 7: einen erfindungsgemäßen Kristallhabitus von Acrylsäure aus einem 90/10 w/w Acrylsäure-Wassergemisch;
- Fig. 8: einen erfindungsgemäßen Kristallhabitus von Acrylsäure aus einem 80/20 w/w Acrylsäure-Wassergemisch; und
- Fig. 9: einen erfindungsgemäßen Kristallhabitus von Acrylsäure aus einem 70/30 w/w Acrylsäure-Wassergemisch.

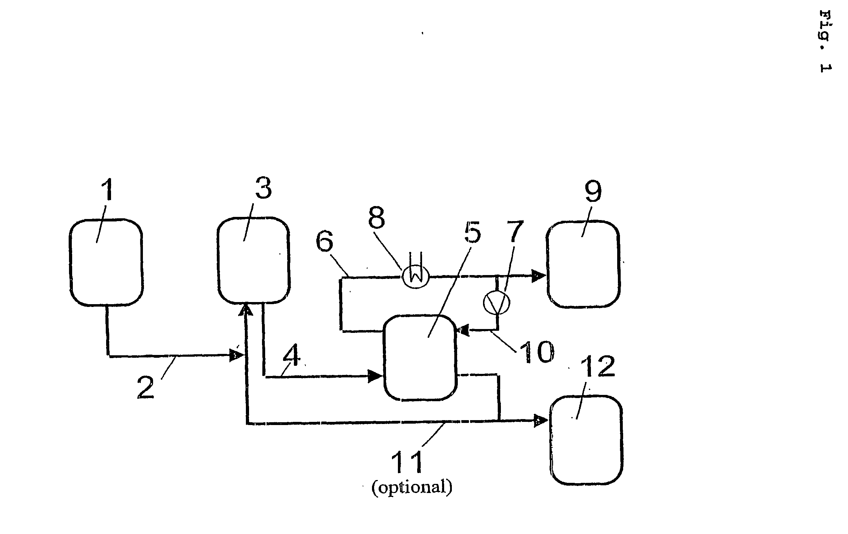

Die Figur 1 zeigt ein Verfahrensschema, bei welchem das Verfahren (in einer Stufe) zusammenfassend beschrieben wird:

### Stufe 1:

1. Vorratsbehälter
2. Zulauf 1 in 3
3. Suspensionserzeuger (z.B. Kühlscheibenkristallisator, Kratzkühler)
4. Zulauf 3 in 5
5. Waschkolonne, hydraulisch oder mechanisch
6. Produktkreislauf
7. Produktkreislaufpumpe
8. Produktkreislaufwärmetauscher als Aufschmelzer
9. Produktbehälter
10. Produktrückführung zur Gegenstromwäsche in der Waschkolonne 5
11. Mutterlaugenrückführung (optional)
12. Mutterlaugenbehälter (Abstoss)

Die Waschkolonne 5, der Suspensionserzeuger 3, der Produktkreislaufwärmetauscher 8, der Zulauf 4, der Produktkreislauf 6 und die Produktrückführung 10 entsprechen dem Trennbereich, dem Kristallisationsbereich, dem Aufschmelzer, der ersten Führung, der zweiten Führung, der vierten beziehungsweise, wenn in der Waschkolonne Kristallbildung stattfindet, der dritten Führung.

Aus dem Vorratsbehälter 1 wird die zu trennende Schmelze im flüssigen Zustand, also knapp über Gleichgewicht temperiert über den Zulauf 2 in den Suspensionserzeuger 3 geführt. Durch Kühlung unter die Gleichgewichtstemperatur der Schmelze entstehen im Suspensionserzeuger 3 kontinuierlich Kristalle mit einer Suspensionsdichte zwischen 5 und 50% (vorzugsweise 20 bis 30%). Die Suspension wird kontinuierlich über den Zulauf 4 in die Waschkolonne 5 geführt und dort über bewegte oder feststehende Filter (hydraulisch oder mechanisch, s.o.) in eine flüssige und eine feste Phase getrennt. Das Filtrat verläßt die Waschkolonne 5 und wird dem Mutterlaugenbehälter 12 kontinuierlich zugeführt. Zur Ausbeutesteigerung ist es optional möglich, zumindest einen Teil des Filtrats auch über die Mutterlaugenrückführung 11 in den Suspensionserzeuger 3 zurückzuführen.

Die Kristalle in der Waschkolonne 5 werden zu einem Kristallbett verdichtet und je nach Typ der Waschkolonne oben oder unten mit umlaufenden Messern abgeschabt. Die abgeschabten Produktkristalle werden als Suspension im Produktkreislauf 6 mit der Kreislaufpumpe 7 umgepumpt und schmelzen durch das Einbringen der Schmelzenthalpie mit dem Aufschmelzer oder Produktwärmetauscher 8 auf.

Ein Teil wird als Waschflüssigkeit zur Gegenstromwäsche in die Waschkolonne durch die Produktrückführung 10 zurückgeführt. Der andere Teil verläßt die Anlage als Produkt in den Behälter 9.

Ist bei dieser einstufigen Fahrweise ein Minimum an Produktverlust durch den Abstoß nicht zu erreichen, kann die Mutterlauge aus der ersten Kristallisationsstufe optional in einer oder mehreren weiteren Kristallisationsstufen oder mit anderen Reinigungsmitteln aufgearbeitet werden.

Figur 2 zeigt exemplarisch die Durchführung in mehreren Kristallisationsstufen

### Stufe 2/3 etc.:

13. Mutterlaugenbehälter der 1. Stufe (=12)
14. Zulauf aus 13 in 15
15. Suspensionserzeuger Stufe 2 (z.B. Kühlscheibenkristallisator, Kratzkühler)
16. Zulauf aus 15 in 17
17. Waschkolonne, hydraulisch oder mechanisch
18. Produktkreislauf der 2. Stufe
19. Produktkreislaufpumpe
20. Produktkreislaufwärmetauscher als Aufschmelzer
22. Produktrückführung in 17
21. Mutterlaugenrückführung in den Kristaller der 2. Stufe (optional)
22. Mutterlaugenbehälter der 2. Stufe (Abstoss)
23. Mutterlaugenzuführung aus der 2. Stufe in 26
24. Suspensionserzeuger Stufe 3 (z.B. Kühlscheibenkristallisator, Kratzkühler
25. Zuführung des Produktes aus 26 in 28
26. Waschkolonne, hydraulisch oder mechanisch
27. Produktkreislauf der 3. Stufe
28. Produktkreislaufpumpe
29. Produktkreislaufwärmetauscher als Aufschmelzer
30. Produktrückführung in 28
31. Mutterlaugenrückführung in den Kristaller der 3. Stufe (optional)
32. Zulauf aus 28 in 35
33. Mutterlaugenbehälter (Abstoß) der 3. Stufe
34. ggf. Zuführung zu weiteren Reinigungsstufen

Der Suspensionserzeuger 15, die Waschkolonne 17, der Produktkreislaufwärmetauscher 20, der Zulauf 16, der Produktkreislauf 18, Produktrückführung 22, die Mutterlaugenrückführung 23 entsprechen jeweils bei der ersten Vorrichtungseinheit dem Kristallisationsbereich, dem Trennbereich und dem Aufschmelzer, der ersten Führung, der zweiten Führung, der dritten bzw. vierten Führung, der zweiten Rückführung.

Der Suspensionserzeuger 26, die Waschkolonne 28, und der Produktkreislaufwärmetauscher 31 der Produktkreislauf 32, die Produktrückführung 29, die Mutterlaugenrückführung 33 entsprechen jeweils bei der zweiten Vorrichtungseinheit dem Kristallisationsbereich, dem Trennbereich, dem Aufschmelzer, der zweiten Führung, der dritten bzw. vierten Führung, der zweiten Rückführung.

Die Mutterlaugenzuführung 25 entspricht einer Verbindungsleitung.

Die Mutterlauge (12/13) aus der 1. Stufe wird teilweise oder vollständig in den Suspensionserzeuger der 2. Stufe 15 geführt. Durch Kühlung unter die Gleichgewichtstemperatur der Schmelze entstehen im Suspensionserzeuger 15 kontinuierlich Kristalle mit einer Suspensionsdichte zwischen 5 und 50% (vorzugsweise 20 bis 30%). Die Suspension wird kontinuierlich über den Zulauf 16 in die Waschkolonne der 2. Stufe (17) geführt und dort über bewegte oder feststehende Filter (hydraulisch oder mechanisch, s.o.) in eine flüssige und eine feste Phase getrennt.

Zu einem Kristallbett verdichtet werden die Kristalle je nach Typ der Waschkolonne 17 oben oder unten mit umlaufenden Messern abgeschabt. Die abgeschabten Produktkristalle werden optional (wie in der 1. Stufe) als Suspension im Produktkreislauf 18 mit der Kreislaufpumpe 19 umgepumpt und schmelzen durch das Einbringen der Schmelzenthalpie mit dem Aufschmelzers 20 auf. Ein Teil kann als Waschflüssigkeit zur Gegenstromwäsche in die Waschkolonne zurückgeführt werden 22. Der andere Teil kann als aufgeschmolzenes Produkt in den Suspensionserzeuger der ersten Stufe 3 zurückgeführt werden. Das Filtrat verläßt die Waschkolonne 17 und wird dem Mutterlaugenbehälter 24 kontinuierlich zugeführt. Zur weiteren Ausbeutesteigerung ist es optional möglich, zumindest einen Teil des Filtrats auch über die Mutterlaugenrückführung 23 in den Suspensionserzeuger 15 zurückzuführen und/oder in einer 3. Stufe weiter aufzuarbeiten.

Dazu wird die Mutterlauge über 25 in einen weiteren Suspensionserzeuger 26 geleitet. Die wie oben gewonnene Suspension wird über 27 in die Waschkolonne 28 geleitet, dort zu einem Kristallbett verdichtet und je nach Typ der Waschkolonne 28 oben oder unten mit umlaufenden Messern abgeschabt. Die abgeschabten Produktkristalle werden optional (wie in der 1. Stufe) als Suspension im Produktkreislauf 29 mit der Kreislaufpumpe 30 umgepumpt und schmelzen durch das Einbringen der Schmelzenthalpie mit dem Wärmetauscher 31 auf.

Ein Teil kann als Waschflüssigkeit zur Gegenstromwäsche in die Waschkolonne zurückgeführt werden 32. Der andere Teil kann als aufgeschmolzenes Produkt in den Suspensionserzeuger der ersten Stufe 3 oder der 2. Stufe 15 zurückgeführt werden.

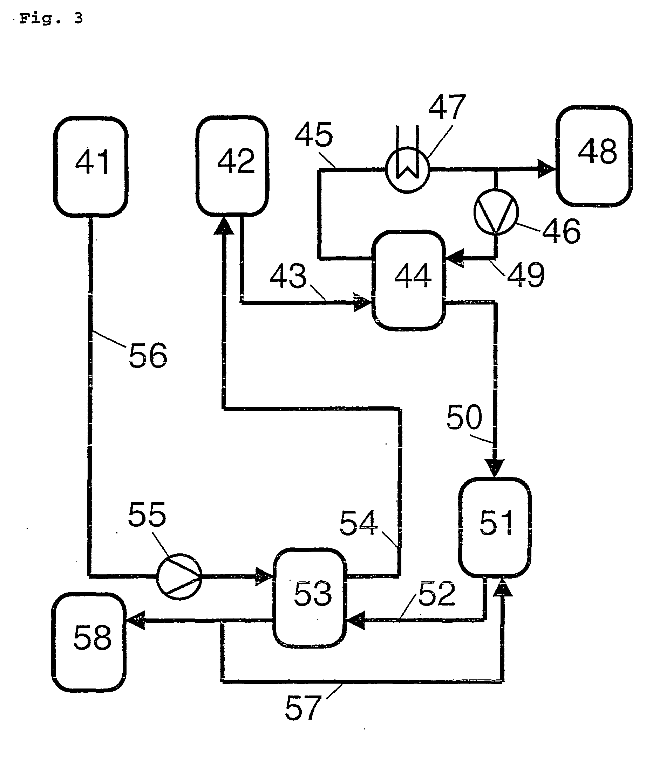

Figur 3 zeigt eine weitere bevorzugte Verschaltung des erfindungsgemäßen Aufarbeitungsverfahrens.
1. Vorratsbehälter
2. Suspensionserzeuger (z.B. Kühlscheibenkristallisator, Kratzkühler)
3. Zulauf 42 in 44
4. Waschkolonne, hydraulisch oder mechanisch
5. Produktkreislauf
6. Produktkreislaufpumpe
7. Produktkreislaufwärmetauscher als Wärmetauscher
8. Produktbehälter
9. Produktrückführung zur Gegenstromwäsche in der Waschkolonne 44
10. Zulauf der 2. Stufe = Mutterlauge aus Stufe 1 (aus der Waschkolonne 44)
11. Suspensionserzeuger Stufe 2 (z.B. Kühlscheibenkristallisator, Kratzkühler)
12. Zulauf 51 in 53
13. Waschkolonne, hydraulisch oder mechanisch
14. Produkt aus der 2. Stufe (Suspension, vermischt mit dem Original aus 41, der nicht in Stufe 1, sondern in Stufe 2 geführt wird), das in den Kratzkühler der 1. Stufe (42) geführt wird
15. Produktzuführungspumpe
16. Zuführung aus der ersten Stufe (41) direkt in den Kopf der Waschkolonne 53 der 2. Stufe
17. Mutterlaugenrückführung in den Kristaller der zweiten Stufe (51) (optional)
18. Mutterlaugenbehälter der zweiten Stufe (Abstoss)

Der Suspensionserzeuger 42, die Waschkolonne 44, der Produktkreislaufwärmetauscher 47, der Produktkreislauf 45, Produktrückführung 49, der Zulauf 43 entsprechen jeweils bei der ersten Vorrichtungseinheit dem Kristallisationsbereich, dem Trennbereich und dem Aufschmelzer, der zweiten Führung, der dritten bzw. vierten Führung, der ersten Führung.

Der Suspensionserzeuger 51, die Waschkolonne 53, der Zulauf 52, die Mutterlaugenrückführung 57 entsprechen jeweils bei der zweiten Vorrichtungseinheit dem Kristallisationsbereich, dem Trennbereich, der ersten Führung, der zweiten Rückführung.

Die Zuläufe 54, 50 entsprechen Verbindungsleitungen. Die hiermit beispielhaft dargestellte Verschachtelung erlaubt vorteilhafterweise, dass die zweite Vorrichtungseinheit keinen Aufschmelzer benötigt.

Eine besonders bevorzugte Variante einer zwei- oder mehrstufigen Ausführung führt die zu trennende Schmelze der ersten Stufe 41 über die Zuführung 56 und die Pumpe 55 in den Kopf der Waschkolonne der 2. Stufe 53 um dort die abgeschabten Produktkristalle der zweiten Stufe als Suspension 54 in den Suspensionserzeuger der 1. Stufe 42 zu führen. Diese Variante hat den energetischen Vorteil, auf ein Aufschmelzen in der zweiten Stufe verzichten zu können und die nun in der ersten Stufe vorhandenen Kristalle nicht noch einmal ausfrieren zu müssen.

Das Gegenstromwaschen in der Waschkolonne der 2. Stufe 53 wird hier durch die Schmelze aus 41 über Zulauf 56 der 1. Stufe erreicht, der im Vergleich zur Verunreinigungskonzentration in der 2. Stufe sehr rein ist und von daher ein ähnlich effektives Waschen wie ein Waschen mit Produkt ermöglicht. Obgleich eine Kombination aus einem Kratzkühler und einer Waschkolonne zur Herstellung organischer Substanzen mit hoher Reinheit bekannt ist, war nicht vorhersehbar, dass dieses Verfahren auch zur Konzentrierung von (Meth)Acrylsäure mit Ausgangsreinheiten von <99,5 Gew.-% und mit hohem Wassergehalt, bezogen auf die Menge Acrylsäure, sehr gut geeignet ist. Überraschend war ferner, dass der Gehalt an Verunreinigungen, wie weitere organische Kohlenstoffverbindungen, durch das erfindungsgemäße Verfahren auf Werte erheblich gesenkt werden kann. Dergestalt können gerade auch kritische Größen wie Fufural, Inhibitoren, Essigsäure oder Maleinsäure auf unkritische Werte abgereichert werden. Dies ist so dem Stand der Technik nicht zu entnehmen gewesen bzw. wird durch diesen auch nicht nahegelegt.

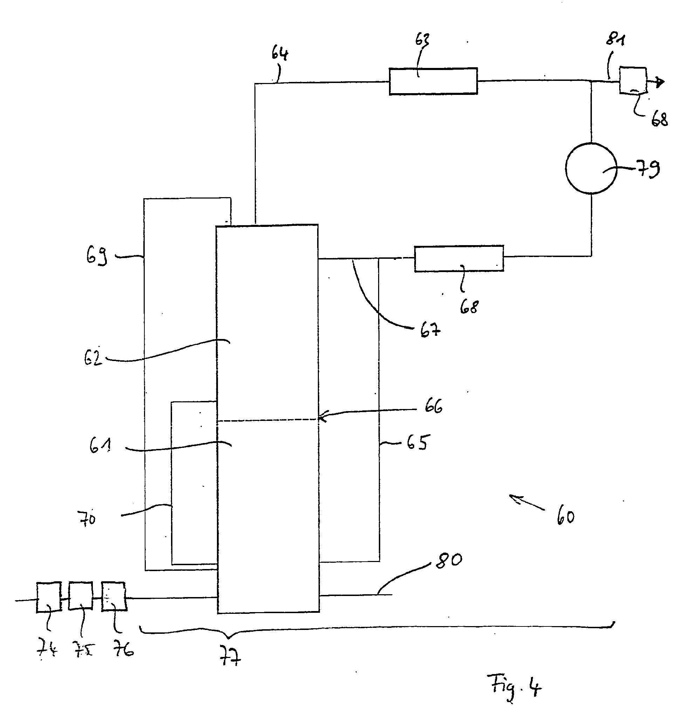

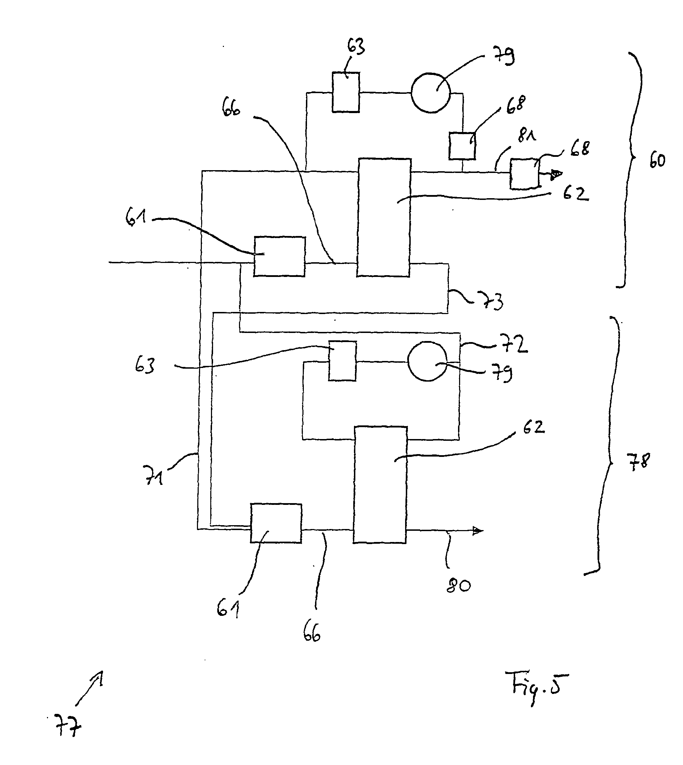

Die Fig. 4 zeigt ein erfindungsgemäßes Verfahrensschema mit einer Verfahrensstufe und die Fig. 5 zeigt ein erfindungsgemäßes Verfahrensschema mit zwei Verfahrensstufen. 60. Vorrichtungseinheit (1. Verfahrensstufe)
61. Kristallisationsbereich
62. Trennbereich
63. Wärmetauscher als Aufschmelzer
64. zweite Führung
65. dritte Führung
66. erste Führung
67. vierte Führung
68. separate Reinigungsvorrichtung
69. erste Rückführung
70. zweite Rückführung
71. Verbindungsleitung
72. Verbindungsleitung
73. Verbindungsleitung
74. (Meth)Acrylsäure-Reaktor
75. Quenchabsorber
76. Destillationsvorrichtung
77. Aufreinigungsvorrichtung
78. Vorrichtungseinheit (2. Verfahrensstufe)
79. Pumpe
80. Restmutterlaugenablauf
81. Produktablauf

Fig. 4 zeigt eine erfindungsgemäße Vorrichtung zur Herstellung von (Meth)Acrylsäure mit einem (Meth)Acrylsäure-Reaktor 74, einem Quenchabsorber 75, einer Destillationsvorrichtung 76 und einer Aufreinigungsvorrichtung 77, wobei die Aufreinigungsvorrichtung 77 einen Kristallisationsbereich 61 und einen Trennbereich 62 enthält. Der Kristallisationsbereich 61 und der Trennbereich 62 liegen eng beieinander, vorzugsweise sind der Kristallisationsbereich 61 und der Trennbereich 62 nicht über Rohrleitungen, sondern direkt miteinander verbunden. Vorzugsweise findet in einem einzigen Gehäuse während des Waschens der Kristalle, insbesondere der Abtrennung der Kristalle von der Mutterlauge, auch ein Kristallwachstum statt. Der Kristallisationsbereich 61 und der Trennbereich 62 sind durch eine erste Führung 66, die durch das gemeinsame Gehäuse gebildet sein kann, miteinander verbunden. Der Trennbereich 62 ist über eine zweite Führung 74 mit dem Aufschmelzer 63 verbunden, der die im Abtrennbereich von der Mutterlauge abgetrennten (Meth)Acrylsäurekristalle aufschmilzt. Die aufgeschmolzene (Meth)Acrylsäure wird mit Hilfe einer Pumpe 79 entweder über eine vierte Führung 67 zum Trennbereich 62 oder über eine dritte Führung 65 zum Kristallisationsbereich 61 befördert. Vorteilhafterweise sorgt eine separate Reinigungsvorrichtung 68 für eine Steigerung der Reinheit der (Meth)Acrylsäure. Um die Kristallisation im Kristallisationsbereich 61 zu fördern werden mit Hilfe einer ersten Rückführung 69 im Trennbereich 62 abgetrennt (Meth)Acrylsäurekristalle als Impfkristalle in den Kristallisationsbereich 61 zugeführt. Die Ausbeute wird gesteigert, in dem die bei einer unvollständigen Abtrennung im Trennbereich 62 in der Mutterlauge verbliebene (Meth)Acrylsäure zurückgewonnen wird, indem die Mutterlauge aus dem Trennbereich 62 über eine zweite Rückführung 70 dem Kristallisationsbereich 61 zugeführt wird. Mit Hilfe eines Produktablaufes 81 wird die gereinigte (Meth)Acrylsäure dem Kreislauf entnommen. Mittels eines Restmutterlaugenablaufes 80 wird nicht mehr verwertbare Restmutterlauge entnommen.

Die Figur 5 zeigt eine Aufreinigungsvorrichtung 77 mit zwei Vorrichtungseinheiten 60, 78. Damit umfasst die Aufreinigungsvorrichtung 77 zwei Verfahrenstufen. Um die erzielbare Reinheit der (Meth)Acrylsäure sowie die Ausbeute zu steigern werden die beiden Vorrichtungseinheiten 60 mit Verbindungsleitungen 71, 72, 73 miteinander verbunden.

In Bezug auf die Mutterlauge, welche von der ersten Vorrichtungseinheit 60 über eine Verbindungsleitung 73 der zweiten Vorrichtungseinheit 78 zuführt wird, sind die beiden Vorrichtungseinheiten 60 und 78 in Reihe verschaltet.

Um die Reinheit der aus der zweiten Verfahrenstufe mit der zweiten Vorrichtungseinheit 78 gewonnenen (Meth)Acrylsäure zu steigern, die aufgrund der niedrigeren (Meth)Acrylsäurekonzentration in der Mutterlauge, die der zweiten Vorrichtungseinheit 78 zugeführt wird, niedriger ausfallen kann, wird zu weiteren Aufreinigung die in der zweiten Verfahrenstufe gewonnene (Meth)Acrylsäure über eine Verbindungsleitung 72 dem Kristallisationsbereich 61 der ersten Vorrichtungseinheit 60 zugeführt. Durch diese Nachreinigung wird ein verbesserte Reinheit der gewonnen (Meth)Acrylsäure gewährleistet.

Im Bezug auf den Transport der Mutterlauge folgt die zweite Vorrichtungseinheit 78 der ersten Vorrichtungseinheit 60. In Bezug auf den Transport der (Meth)Acrylsäure folgt die erste Vorrichtungseinheit 60 der zweiten Vorrichtungseinheit 78. Damit sind die beiden Vorrichtungseinheiten 78 und 60 miteinander verschachtelt.

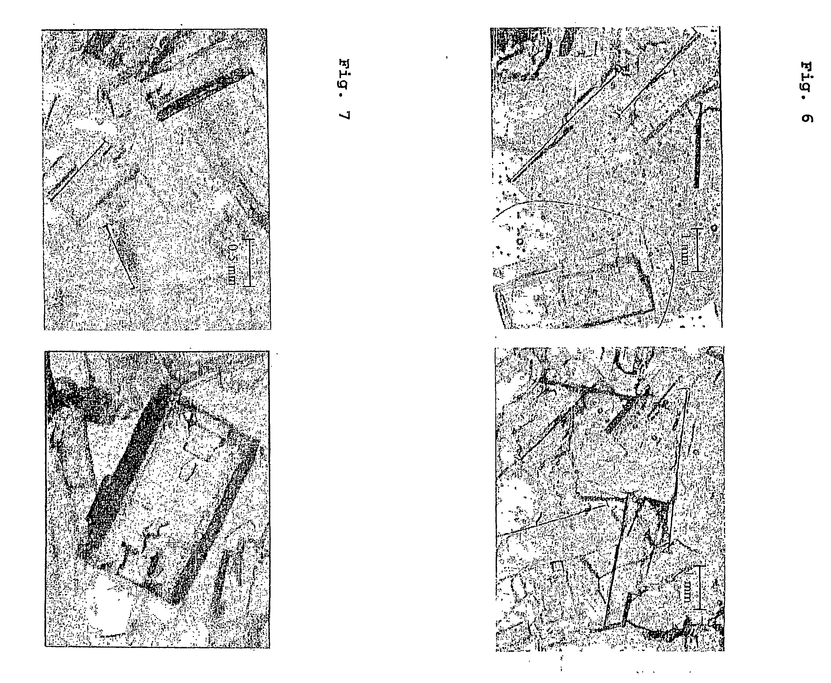

Fig. 6 zeigt einen Kristallhabitus reiner Acrylsäure bei einer Gleichgewichtstemperatur von 12,7°C und einer Kristallisationstemperatur von 11,25°C mit einer Kristallisationszeit von 2h.

Fig. 7 zeigt einen erfindungsgemäßen Kristallhabitus von Acrylsäure aus einem 90/10 w/w Acrylsäure-Wassergemisch mit einer Gleichgewichtstemperatur von 4,5°C mit einer Starttemperatur von 3,1°C und einer Kühlrate von 1K/h mit einer Kristallisationszeit nach 1,5 h. Wie der Vergleich mit von Fig. 6 und Fig. 7 zeigt, weisen die erfindungsgemäßen Kristalle zwar auch eine kleine Anzahl von Einschlüssen auf, der Habitus wird aber vielmehr dahingehend verändert, dass an der jeweiligen Oberseite der Kristalle Ausnehmungen entstehen, aus denen Mutterlauge vorteilhaft abfließen kann. Man erkennt, dass noch nicht alle Kristalle von diesem Phänomen betroffen sind.

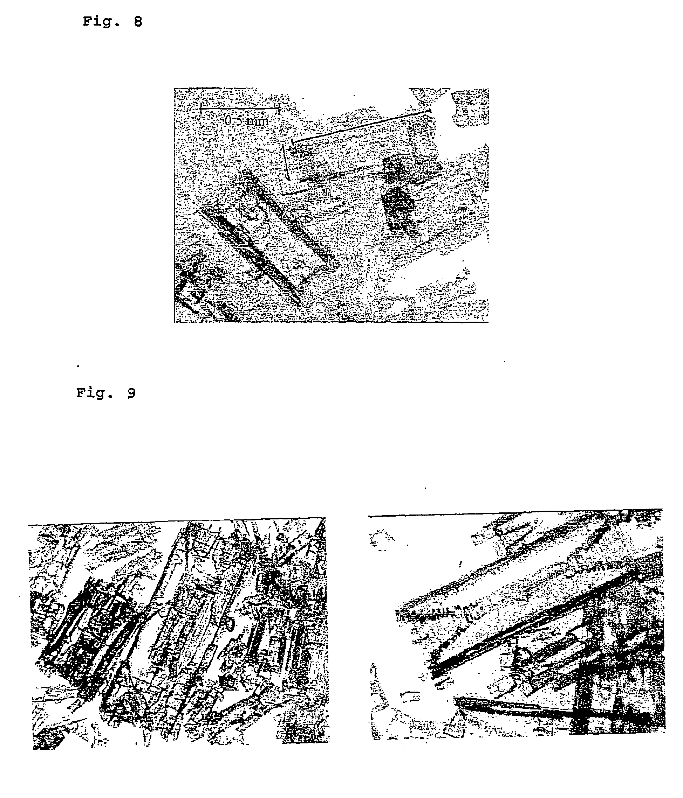

Fig. 8 zeigt einen erfindungsgemäßen Kristallhabitus von Acrylsäure aus einem 80/20 w/w Acrylsäure-Wassergemisch bei einer Gleichgewichtstemperatur von - 2°C und einer Starttemperatur von -3,5°C mit einer Kühlrate von 1K/h und einer Kristallisationszeit von 1,5h. Man erkennt, dass alle Kristalle betroffen sind und die Größe der Einbuchtungen zu nimmt.

Fig. 9 zeigt einen erfindungsgemäßen Kristallhabitus von Acrylsäure aus einem 70/30 w/w Acrylsäure-Wassergemisch bei einer Gleichgewichtstemperatur von - 7,5°C °C und einer Starttemperatur von -8,7°C mit einer Kühlrate von 1K/h und einer Kristallisationszeit von 1,5h. Abb. 5. Man erkennt, dass allgemeine Kristalldefekte zunehmen, wobei der eigentliche Habitus noch gut zu erkennen ist. Die Ausnehmungen sind z.T. so groß, dass die Kristalle innen hohl werden. Auch in einem solchen Fall ist das Abfließen von Mutterlauge gewährleistet und das Auswaschen weiterer Verunreinigungen wird ermöglicht.

Die Erfindung wird nun anhand nicht limitierender Beispiele näher erläutert.

### Beispiel I

### Kristallisation von wasserarmer Acrylsäure

In einer Vorrichtung entsprechend der Figur 1 mit einem Kratzkristaller zur Suspensionserzeugung und einer mechanischen Waschkolonne mit unten angeordnetem Kolben und oben angeordnetem Abzug der gereinigten Schmelze wurde Acrylsäure mit folgender Zusammensetzung (Tabelle 1) in den Kratzkühler vorgelegt.

**Tabelle 1**

| Name | | Vorlage |
|---|---|---|
| Farbzahl | - | >700 |
| Wasser | % | 13,17 |
| Essigsäure | % | 1,304 |
| Furfural | % | 0,033 |
| Benzaldehyd | % | 0,038 |
| Propionsäure | % | 0,018 |
| Acrolein | % | 0,012 |
| Protoanemonin | % | 0,024 |
| Acrylsäure | % | 85,140 |
| Hydrochinon | % | 0,028 |
| Phenothiazin | % | 0,008 |
| Dimere-Acrylsäure | % | 0,144 |
| Maleinsäure-anhydrid | % | 0,276 |
| Sonstige | % | - |

Als Feed (wasserarme Zusammensetzung) wurde Acrylsäure derselben in Tabelle 1 dargestellten Zusammensetzung verwendet.

Der Kratzkühler wurde abgekühlt, wobei es im Kratzkühler bei ca. -5°C zur Ausbildung einer Kristallschicht kam, die durch die umlaufenden Schaber im Kratzkühler zur Bildung einer Suspension abgeschabt wurde. Die abfiltrierte Mutterlauge wurde stets vollständig aus dem Prozess geschleust. Nach ca. 12 stündigem Betrieb wies die flüssige Phase im Kristaller (=Filtrat aus der Waschkolonne) die Zusammensetzung nach Tabelle 2 auf.

**Tabelle 2**

| Name | | Filtrat |
|---|---|---|
| Farbzahl | - | >700 |
| Wasser | % | 20,846 |
| Essigsäure | % | 1,855 |
| Furfural | % | 0,045 |
| Benzaldehyd | % | 0,078 |
| Propionsäure | % | 0,011 |
| Acrolein | % | 0,042 |
| Protoanemonin | % | 0,032 |
| Acrylsäure | % | 75,991 |
| Hydrochinon | % | 0,112 |
| Phenothiazin | % | 0,013 |
| Dimere-Acrylsäure | % | 0,467 |
| Maleinsäure-anhydrid | % | 0,301 |
| Sonstige | % | 0,208 |

Die aus dem Kristaller abgezogene Kristallsuspension wurde in der Waschkolonne zu einem kompakten Kristallbett verdichtet. An der Oberseite des Kristallbetts wurde dieses mittels eines rotierenden Schabers abgeschabt, als Kristallsuspension im Produktkreislauf umgepumpt und durch den Wärmetauscher aufgeschmolzen. Ein Teil wurde zur Gegenstromwäsche in das Kristallbett zurückgeführt, um die Waschfront konstant zu halten, damit weder Produkt noch Mutterlauge durch das Kristallbett schlägt. Der andere Teil wurde kontinuierlich als Produkt gewonnen. Exemplarisch für die erreichten Produktqualitäten sind in der Tabelle 3 die Mittelwerte der Produktanalyse derjenigen Acrylsäure genannt, die ca. 1-3 Stunden später als die Probe aus Tabelle 2 genommen wurde, also ca. 12 bis 15 Stunden nach Versuchsbeginn.

**Tabelle 3**

| Name | | Produkt |
|---|---|---|
| Farbzahl | - | 10 |
| Wasser | % | 0,043 |
| Essigsäure | % | 0,182 |
| Furfural | % | <0,0001 |
| Benzaldehyd | % | <0,0001 |
| Propionsäure | % | 0,006 |
| Acrolein | % | <0,0001 |
| Protoanemonin | % | <0,0001 |
| Acrylsäure | % | 99,764 |
| Hydrochinon | % | <0,001 |
| Phenothiazin | % | <0,0001 |
| Dimere-Acrylsäure | % | 0,006 |
| Maleinsäure-anhydrid | % | <0,005 |
| Sonstige | % | - |

Wie aus Tabelle 3 ersichtlich, ermöglicht das erfindungsgemäße Verfahren die Herstellung hochreiner Acrylsäure.

Die Konzentrationsangaben wurden per GC ermittelt. Die Farbzahl nach der Methode DIN-ISO 6271. Wasser wurde nach ASTM D 1364 und die Inhibitoren (MEHQ) nach ASTM D 3125 bestimmt.

### Beispiel II

### Kristallisation von wässeriger Acrylsäure

In ein gerührtes Doppelmantelgefäß wurden 760g einer Probe eines Quenchabsorbersumpfes einer Acrylsäureanlage mit einer Zusammensetzung nach Tabelle 4 vorgelegt und durch einen Kryostat abgekühlt.

**Tabelle 4**

| Name | | Feed |
|---|---|---|
| Wasser | % | 32,7 |
| Hydrochinon | % | 0,059 |
| Essigsäure | % | 3,617 |
| Furfural | % | 0,0235 |
| Benzaldehyd | % | 0,0269 |
| Propionsäure | % | 0,012 |
| Acrolein | % | 0,0101 |
| Protoanemin | % | 0,0161 |
| Acrylsäure | % | 62,586 |
| D-Acrylsäure | % | 1,088 |
| MSA | % | 0,042 |
| Sonstige | % | 0,269 |
| Ges. Nebenk. | % | 4,714 |

Die vorgelegte Probe wurde auf 11°C und danach mit einer Kühlrate mit 0,1K/min weiter abgekühlt. Bei -15°C wurde mit 1mg Eis und 1mg kristalliner Acrylsäure angeimpft. Daraufhin erfolgte eine Trübung der Lösung. Nach weiteren 10 Minuten wurde die Suspension auf einer auf 0°C temperierten Vakuum-Saugnutsche mit 250µm-Propyltex-Filtergewebe Fest-Flüssig getrennt. Die dabei entstandene Mutterlauge (Filtrat, 380g) weist eine Zusammensetzung nach Tabelle 5 auf. Diese Mutterlauge ist an Acrylsäure stärker verarmt als an Wasser.

**Tabelle 5**

| Name | | Filtrat |
|---|---|---|
| Wasser | % | 36,9 |
| Hydrochinon | % | 0,072 |
| Essigsäure | % | 3,379 |
| Furfural | % | 0,0251 |
| Benzaldehyd | % | 0,0308 |
| Propionsäure | % | 0,010 |
| Acrolein | % | 0,0113 |
| Protoanemin | % | 0,0170 |
| Acrylsäure | % | 57,984 |
| D-Acrylsäure | % | 1,047 |
| MSA | % | 0,035 |
| Sonstige | % | 0,489 |
| Ges. Nebenk. | % | 5,1162 |

Auf der Saugnutsche wurden zwei verschiedene Kristallhabiti (einmal klar weiß und einmal milchig) mit unterschiedlichen Schmelztemperaturen und Schmelzverhaltens beobachtet. Die 214g restfeuchten Kristalle auf der Saugnutsche zeigt die Zusammensetzung nach Tabelle 6.

**Tabelle 6**

| Name | | Kristalle vor dem Waschen (filterfeucht) |
|---|---|---|
| Wasser | % | 23,4 |
| Hydrochinon | % | 0,039 |
| Essigsäure | % | 2,364 |
| Furfural | % | 0,0161 |
| Benzaldehyd | % | 0,0183 |
| Propionsäure | % | 0,009 |
| Acrolein | % | 0,0092 |
| Protoanemin | % | 0,0107 |
| Acrylsäure | % | 72,926 |
| D-Acrylsäure | % | 0,799 |
| MSA | % | nicht messbar |
| Sonstige | % | 0,409 |
| Ges. Nebenk. | % | 3,674 |

Die filterfeuchten Kristalle nach Tabelle 6 wurden mit einer Menge von 200g auf 0°C temperierten VE-Wasser gewaschen. Der so erhaltene Filterkuchen zeigt die in Tabelle 7 aufgeführte Zusammensetzung.

**Tabelle 7**

| Name | | Kristalle vor dem Waschen (filterfeucht) |
|---|---|---|
| Wasser | % | 38,8 |
| Hydrochinon | % | 0,010 |
| Essigsäure | % | 1,005 |
| Furfural | % | 0,0055 |
| Benzaldehyd | % | 0,0067 |
| Propionsäure | % | 0,004 |
| Acrolein | % | 0,0024 |
| Protoanemin | % | 0,0037 |
| Acrylsäure | % | 59,707 |
| D-Acrylsäure | % | 0,393 |
| MSA | % | 0,014 |
| Sonstige | % | 0,048 |
| Ges. Nebenk. | % | 1,493 |

Die vorstehenden Mengenangaben wurden gaschromatographisch bestimmt.

In diesem Beispiel wurde eine signifikante Abreicherung der die wässrig Acrylsäure begleitenden Nebenprodukte aus der Synthese der Acrylsäure erreicht, wie sich aus dem Vergleich der Gesamt-Nebenkomponenten (Ges. Nebenk.) ergibt.

## Patentansprüche

1. Verfahren zur Aufreinigung von einer Zusammensetzung beinhaltend (Meth)Acrylsäure, mindestens eine Verunreinigung und Wasser, wobei die Zusammensetzung einen Wassergehalt im Bereich von 0,55 bis 90, bezogen auf die Zusammensetzung, aufweist zu einer gereinigten Phase, beinhaltend (Meth)Acrylsäure und mindestens eine Verunreinigung, wobei in der gereinigten Phase die Menge an mindestens einer Verunreinigung weniger als 7 Gew.-%, bezogen auf die (Meth)Acrylsäure in der gereinigten Phase, beinhaltet, aufweisend eine Verfahrensstufe, welche folgende Verfahrensschritte umfasst:
a) (Meth)Acrylsäure wird aus der Zusammensetzung unter Bildung einer Suspension, beinhaltend eine Mutterlauge und (Meth)Acrylsäurekristalle, auskristallisiert;
b) (Meth)Acrylsäurekristalle werden von der Mutterlauge abgetrennt;
c) mindestens ein Teil der abgetrennten (Meth)Acrylsäurekristalle wird zu einer Schmelze aufgeschmolzen; und
d) ein Teil der Schmelze wird dem Schritt a) oder dem Schritt b) zurückgeführt und wobei der nicht zurückgeführte Teil der Schmelze als eine abgetrennte (Meth)Acrylsäure vorliegt.

2. Verfahren nach Anspruch 1, wobei in dem Schritt a) (Meth)Acrylsäure zumindest teilweise zu einem Kristall mit einer Kristallstruktur mit einer Oberfläche mit mindestens einer an der Oberfläche befindlichen Ausnehmung auskristallisiert, wobei die Kristallstruktur ein orthorombisches Bravais-Kristallgitter mit Raumgruppe Ibam, kristallographische Daten a=9,952 A, b=11,767 A und c=6,206 A aufweist.

3. Verfahren nach einem der Ansprüche 1 oder 2 wobei in dem Schritt a) die Mutterlauge zumindest 60 Gew.-% (Meth)Acrylsäure und Wasser aufweist, wobei die Wasserkonzentration der Mutterlauge im Bereich von 10 und 90 Gew.-% liegt.

4. Verfahren nach einem der vorhergehenden Ansprüche, wobei die (Meth)Acrylsäurekristalle im Gegenstrom von der zurückgeführten Schmelze gewaschen werden.

5. Verfahren nach einem der vorhergehenden Ansprüche, wobei die Schmelze in einem separaten Reinigungsverfahren gereinigt wird.

6. Verfahren nach einem der vorhergehenden Ansprüche, wobei die (Meth)Acrylsäurekristalle aus Schritt b) zumindest teilweise in den Schritt a) zugeführt werden.

7. Verfahren nach einem der vorhergehenden Ansprüche, wobei die nach dem Schritt b) abgetrennte Mutterlauge zumindest teilweise in den Schritt a) zurückgeführt wird.

8. Verfahren nach einem der vorhergehenden Ansprüche, wobei das Verfahren mindestens zwei Verfahrensstufen aufweist, die jeweils die Schritte a) bis d) aufweisen, wobei mindestens eines der folgenden Merkmale (α1) bis (α4) erfüllt ist:
(α1) abgetrennte (Meth)Acrylsäure aus einer ersten Verfahrensstufe wird zumindest teilweise einer zweiten Verfahrensstufe zugeführt;
(α2) abgetrennte (Meth)Acrylsäure aus einer zweiten Verfahrensstufe wird zumindest teilweise einer ersten Verfahrensstufe zugeführt;
(α3) Mutterlauge einer ersten Verfahrensstufe wird zumindest teilweise einer zweiten Verfahrensstufe zugeführt;
(α4) Mutterlauge einer zweiten Verfahrensstufe wird zumindest teilweise einer ersten Verfahrensstufe zugeführt.

9. Vorrichtung zur Herstellung von (Meth)Acrylsäure umfassend als fluidleitend miteinander verbundene Komponenten eine (Meth)Acrylsäure-Syntheseeinheit, eine Destillationsvorrichtung und eine Aufreinigungsvorrichtung, die eine Vorrichtungseinheit aufweist, welche die Merkmale (δ1) bis (δ4) umfasst:
(δ1) die Vorrichtungseinheit weist einen Kristallisationsbereich, einen Trennbereich, einen Aufschmelzer, und mindestens drei Führungen auf;
(δ2) der Kristallisationsbereich ist mit dem Trennbereich über eine erste Führung verbunden;
(δ3) der Trennbereich ist mit dem Aufschmelzer über eine zweite Führung verbunden;
(δ4) der Aufschmelzer ist mit dem Kristallisationsbereich über eine dritte Führung oder mit dem Trennbereich über eine vierte Führung verbunden;
wobei die Aufreinigungsvorrichtung einen Zulauf aufweist, der eine Zusammensetzung, beinhaltend (Meth)Acrylsäure, mindestens eine Verunreinigung und Wasser, wobei die Zusammensetzung einen Wassergehalt im Bereich von 0,55 bis 90, vorzugsweise von 7 bis 50 und besonders bevorzugt von 10 bis 25 Gew.-%, bezogen auf die Zusammensetzung, aufweist, führt.

10. Vorrichtung nach Anspruch 9, wobei die (Meth)Acrylsäuresyntheseeinheit und die Aufreinigungsvorrichtung ohne eine Destillationsvorrichtung miteinander verbunden sind.

11. Vorrichtung nach einem der Ansprüche 9 oder 10, wobei die Vorrichtungseinheit eine separate Reinigungsvorrichtung aufweist.

12. Vorrichtung nach einem der Ansprüche 9 bis 11, wobei der Trennbereich mit dem Kristallisationsbereich durch eine erste Rückführung für abgetrennte (Meth)Acrylsäure verbunden ist.

13. Vorrichtung nach einem der Ansprüche 9 bis 12, wobei der Trennbereich mit dem Kristallisationsbereich durch eine zweite Rückführung für abgetrennte Mutterlauge verbunden ist.

14. Vorrichtung nach einem der Ansprüche 9 bis 13, **gekennzeichnet durch** mindestens zwei Vorrichtungseinheiten gemäß den Merkmalen (δ1) bis (δ4), die **durch** mindestens eine Verbindungsleitung verbunden sind, wobei die Verbindungsleitung eine Zuleitung oder eine Rückleitung ist, und wobei mindestens eines der folgenden Merkmale (ε1) bis (ε4) erfüllt ist:
(ε1) der Trennbereich einer ersten Vorrichtungseinheit ist über die Verbindungsleitung mit dem Kristallisationsbereich einer zweiten Vorrichtungseinheit verbunden;
(ε2) der Aufschmelzer einer ersten Vorrichtungseinheit ist über die Verbindungsleitung mit dem Kristallisationsbereich einer zweiten Vorrichtungseinheit verbunden;
(ε3) der Trennbereich einer zweiten Vorrichtungseinheit ist über die Verbindungsleitung mit dem Kristallisationsbereich einer ersten Vorrichtungseinheit verbunden;
(ε4) der Aufschmelzer einer zweiten Vorrichtungseinheit ist über die Verbindungsleitung mit dem Kristallisationsbereich einer ersten Vorrichtungseinheit verbunden.

15. Vorrichtung zur Polymerisation von (Meth)Acrylsäure, beinhaltend eine Vorrichtung zur Herstellung von (Meth)Acrylsäure nach einem der Ansprüche 9 bis 14 und eine Polymerisationseinheit, wobei die Aufreinigunsvorrichtung der Vorrichtung zur Herstellung von (Meth)Acrylsäure mit der Polymerisationseinheit verbunden ist.

16. Verfahren nach einem der Ansprüche 1 bis 8, wobei das Verfahren in einer Vorrichtung nach einem der Ansprüche 10 bis 14 erfolgt.

## Claims

1. Process for purifying a composition comprising (meth)acrylic acid, at least one impurity and water, the composition having a water content in the range from 0.55 to 90% by weight, based on the composition, to give a purified phase comprising (meth)acrylic acid and at least one impurity, the amount of at least one impurity in the purified phase being less than 7% by weight, based on the (meth)acrylic acid in the purified phase, comprising a process stage which comprises the following steps:
a) (meth)acrylic acid is crystallized out of the composition to form a suspension comprising a mother liquor and (meth)acrylic acid crystals;
b) (meth)acrylic acid crystals are removed from the mother liquor;
c) at least some of the (meth)acrylic acid crystals removed are melted to form a melt; and
d) some of the melt is recycled to step a) or to step b), and the unrecycled portion of the melt is present as a removed (meth)acrylic acid.

2. Process according to Claim 1, wherein, in step a), (meth)acrylic acid is at least partly crystallized to give a crystal with a crystal structure with a surface with at least one recess on the surface, the crystal structure having an orthorhombic Bravais crystal lattice of space group Ibam, crystallographic data a = 9.952 A, b = 11.767 A and c = 6.206 A.

3. Process according to either of Claims 1 and 2, wherein, in step a), the mother liquor comprises at least 60% by weight of (meth)acrylic acid and water, the water concentration of the mother liquor being in the range from 10 to 90% by weight.

4. Process according to any of the preceding claims, wherein the (meth)acrylic acid crystals are washed in countercurrent by the recycled melt.

5. Process according to any of the preceding claims, wherein the melt is purified in a separate purification process.

6. Process according to any of the preceding claims, wherein the (meth)acrylic acid crystals from step b) are at least partly fed into step a).

7. Process according to any of the preceding claims, wherein the mother liquor removed after step b) is at least partly recycled into step a).

8. Process according to any of the preceding claims, which has at least two process stages, each of which has steps a) to d), at least one of the following features (α1) to (α4) being satisfied:
(α1) removed (meth)acrylic acid from a first process stage is at least partly fed to a second process stage;
(α2) removed (meth)acrylic acid from a second process stage is at least partly fed to a first process stage;
(α3) mother liquor of a first process stage is at least partly fed to a second process stage;
(α4) mother liquor of a second process stage is at least partly fed to a first process stage.

9. Apparatus for preparing (meth)acrylic acid, comprising, as components with a fluid-conducting connection to one another, a (meth)acrylic acid synthesis unit, a distillation apparatus and a purification apparatus which has an apparatus unit which comprises features (δ1) to (δ4):
(δ1) the apparatus unit has a crystallization region, a separation region, a melting apparatus and at least three conduits;
(δ2) the crystallization region is connected to the separation region via a first conduit;
(δ3) the separation region is connected to the melting apparatus via a second conduit;
(δ4) the melting apparatus is connected to the crystallization region via a third conduit, or to the separation region via a fourth conduit;
the purification apparatus having a feed which conducts a composition comprising (meth)acrylic acid, at least one impurity and water, the composition having a water content in the range from 0.55 to 90, preferably 7 to 50 and more preferably 10 to 25% by weight, based on the composition.

10. Apparatus according to Claim 9, wherein the (meth)acrylic acid synthesis unit and the purification apparatus are connected to one another without a distillation apparatus.

11. Apparatus according to either of Claims 9 and 10, wherein the apparatus unit has a separate purifying apparatus.

12. Apparatus according to any of Claims 9 to 11, wherein the separation region is connected to the crystallization region by a first recycle line for removed (meth)acrylic acid.

13. Apparatus according to any of Claims 9 to 12, wherein the separation region is connected to the crystallization region by a second recycle line for removed mother liquor.

14. Apparatus according to any of Claims 9 to 13, **characterized by** at least two apparatus units according to features (δ1) to (δ4) which are connected by at least one connecting line, the connecting line being a feed line or a return line, and at least one of the following features (ε1) to (ε4) being satisfied:
(ε1) the separation region of a first apparatus unit is connected via the connecting line to the crystallization region of a second apparatus unit;
(ε2) the melting apparatus of a first apparatus unit is connected via the connecting line to the crystallization region of a second apparatus unit;
(ε3) the separation region of a second apparatus unit is connected via the connecting line to the crystallization region of a first apparatus unit;
(ε4) the melting apparatus of a second apparatus unit is connected via the connecting line to the crystallization region of a first apparatus unit.

15. Apparatus for polymerizing (meth)acrylic acid, comprising an apparatus for preparing (meth)acrylic acid according to any of Claims 9 to 14 and a polymerization unit, wherein the purifying apparatus of the apparatus for preparing (meth)acrylic acid is connected to the polymerization unit.

16. Process according to any of Claims 1 to 8, which is effected in an apparatus according to any of Claims 10 to 14.

## Revendications

1. Procédé de purification d'une composition contenant de l'acide (méth)acrylique, au moins une impureté et de l'eau, la composition présentant une teneur en eau dans la plage allant de 0,55 à 90 % en poids, par rapport à la composition, pour former une phase purifiée, contenant de l'acide (méth)acrylique et au moins une impureté, la quantité de la ou des impuretés contenues dans la phase purifiée étant inférieure à 7 % en poids par rapport à l'acide (méth)acrylique dans la phase purifié, qui comporte une étape de procédé qui comprend les étapes de procédé suivantes :
a) l'acide (méth)acrylique est cristallisé à partir de la composition en formant une suspension, qui contient une liqueur mère et des cristaux d'acide (méth)acrylique ;
b) les cristaux d'acide (méth)acrylique sont séparés de la liqueur mère ;
c) au moins une partie des cristaux d'acide (méth)acrylique séparés est fondue pour former une masse fondue ; et
d) une partie de la masse fondue est recyclée dans l'étape a) ou dans l'étape b), la partie non recyclée de la masse fondue se présentant sous la forme d'un acide (méth)acrylique séparé.

2. Procédé selon la revendication 1, dans lequel l'acide (méth)acrylique est cristallisé à l'étape a) pour former au moins partiellement un cristal dont la structure cristalline comporte une surface avec au moins une ouverture se trouvant sur la surface, la structure cristalline présentant un réseau cristallin de Bravais orthorhombique, avec le groupe spatial Ibam et les données cristallographiques a = 9,952 A, b = 11,767 A et c = 6,206 A.

3. Procédé selon l'une quelconque des revendications 1 ou 2, dans lequel la liqueur mère comporte à l'étape a) au moins 60 % en poids d'acide (méth)acrylique et de l'eau, la concentration en eau de la liqueur mère se situant dans la plage allant de 10 à 90 % en poids.

4. Procédé selon l'une quelconque des revendications précédentes, dans lequel les cristaux d'acide (méth)acrylique sont lavés à contre-courant de la masse fondue recyclée.

5. Procédé selon l'une quelconque des revendications précédentes, dans lequel la masse fondue est purifiée lors d'un procédé de purification distinct.

6. Procédé selon l'une quelconque des revendications précédentes, dans lequel les cristaux d'acide (méth)acrylique issus de l'étape b) sont recyclés au moins partiellement dans l'étape a).

7. Procédé selon l'une quelconque des revendications précédentes, dans lequel la liqueur mère séparée selon l'étape b) est recyclée au moins partiellement dans l'étape a).

8. Procédé selon l'une quelconque des revendications précédentes, dans lequel le procédé comporte au moins deux étapes de procédé, qui comportent chacune les étapes a) à d), au moins une des caractéristiques (α1) à (α4) suivantes étant satisfaite :
(α1) l'acide (méth)acrylique séparé par une première étape de procédé est introduit au moins partiellement dans une seconde étape de procédé ;
(α2) l'acide (méth)acrylique séparé par une seconde étape de procédé est introduit au moins partiellement dans une première étape de procédé ;
(α3) la liqueur mère d'une première étape de procédé est introduite au moins partiellement dans une seconde étape de procédé ;
(α4) la liqueur mère d'une seconde étape de procédé est introduite au moins partiellement dans une première étape de procédé.

9. Dispositif de fabrication d'acide (méth)acrylique, comprenant, en tant que composants reliés fluidiquement les uns aux autres, une unité de synthèse d'acide (méth)acrylique, un dispositif de distillation et un dispositif de purification, qui comporte une unité de dispositif qui comprend les caractéristiques (δ1) à (δ4) :
(δ1) l'unité de dispositif comporte une zone de cristallisation, une zone de séparation, un dispositif de fusion et au moins trois conduites ;
(δ2) la zone de cristallisation est reliée à la zone de séparation par une première conduite ;
(δ3) la zone de séparation est reliée au dispositif de fusion par une deuxième conduite ;
(δ4) le dispositif de fusion est relié à la zone de cristallisation par une troisième conduite ou à la zone de séparation par une quatrième conduite ;
le dispositif de purification comportant une alimentation qui conduit une composition contenant de l'acide (méth)acrylique, au moins une impureté et de l'eau, la composition présentant une teneur en eau dans la plage allant de 0,55 à 90, de préférence de 7 à 50 et de manière particulièrement préférée de 10 à 25 % en poids, par rapport à la composition.

10. Dispositif selon la revendication 9, dans lequel l'unité de synthèse d'acide (méth)acrylique et le dispositif de purification sont reliés l'un à l'autre sans dispositif de distillation.

11. Dispositif selon l'une quelconque des revendications 9 ou 10, dans lequel l'unité de dispositif comporte un dispositif de purification distinct.

12. Dispositif selon l'une quelconque des revendications 9 à 11, dans lequel la zone de séparation est reliée à la zone de cristallisation par une première conduite de recyclage pour l'acide (méth)acrylique séparé.

13. Dispositif selon l'une quelconque des revendications 9 à 12, dans lequel la zone de séparation est reliée à la zone de cristallisation par une seconde conduite de recyclage pour la liqueur mère séparée.

14. Dispositif selon l'une quelconque des revendications 9 à 13, **caractérisé par** au moins deux unités de dispositif selon les caractéristiques (51) à (54), qui sont reliées par au moins une conduite de liaison, la conduite de liaison étant une conduite d'alimentation ou une conduite de recyclage, et au moins une des caractéristiques (ε1) à (ε4) suivantes étant satisfaite :
(ε1) la zone de séparation d'une première unité de dispositif est reliée à la zone de cristallisation d'une seconde unité de dispositif par la conduite de liaison ;
(ε2) le dispositif de fusion d'une première unité de dispositif est relié à la zone de cristallisation d'une seconde unité de dispositif par la conduite de liaison ;
(ε3) la zone de séparation d'une seconde unité de dispositif est reliée à la zone de cristallisation d'une première unité de dispositif par la conduite de liaison ;
(ε4) le dispositif de fusion d'une seconde unité de dispositif est relié à la zone de cristallisation d'une première unité de dispositif par la conduite de liaison.

15. Dispositif de polymérisation d'acide (méth)acrylique, contenant un dispositif de fabrication d'acide (méth)acrylique selon l'une quelconque des revendications 9 à 14 et une unité de polymérisation, le dispositif de purification du dispositif de fabrication d'acide (méth)acrylique étant relié à l'unité de polymérisation.

16. Procédé selon l'une quelconque des revendications 1 à 8, dans lequel le procédé a lieu dans un dispositif selon l'une quelconque des revendications 10 à 14.
